(19) Europäisches Patentamt
European Patent Office
Office européen des brevets

(11) Veröffentlichungsnummer: **0 283 762 A1**

# (12) EUROPÄISCHE PATENTANMELDUNG

(21) Anmeldenummer: **88102978.9**

(22) Anmeldetag: **29.02.88**

(51) Int. Cl.[4]: **C07D 277/34**, C07D 277/36, C07D 417/12, A01N 43/78

(30) Priorität: **13.03.87 DE 3708202**

(43) Veröffentlichungstag der Anmeldung: **28.09.88 Patentblatt 88/39**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Schwamborn, Michael, Dr.**
**Von-Lohe-Strasse 9**
**D-5000 Koeln 80(DE)**
Erfinder: **Fischer, Reiner, Dr.**
**Rembrandtstrasse 15**
**D-4019 Monheim(DE)**
Erfinder: **Sasse, Klaus, Dr.**
**Puetzweg 13**
**D-5060 Bergisch Gladbach 2(DE)**
Erfinder: **Hagemann, Hermann, Dr.**
**Kandinskystrasse 52**
**D-5090 Leverkusen 1(DE)**
Erfinder: **Schubart, Rüdiger, Dr.**
**An der Engelsfuhr 27**
**D-5060 Bergisch Gladbach 2(DE)**
Erfinder: **Santel, Hans-Joachim, Dr.**
**Gruenstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Strang, Robert Harry, Dr.**
**Unterdorfstrasse 6A**
**D-4000 Düsseldorf 31(DE)**

(54) **Neue Thiazolylether- und -thioetherderivate.**

(57) Thiazolylderivate der allgemeinen Formel

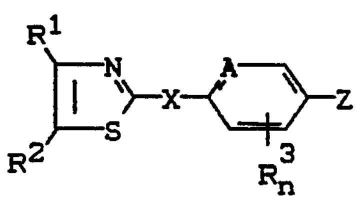

in welcher

EP 0 283 762 A1

$R_1$, $R_2$, $R_3$, A, X, Z und n die in der Beschreibung angegebene Bedeutung haben, Verfahren zu deren Herstellung und ihre Verwendung als Herbizide.

## Neue Thiazolylether-und -thioetherderivate

Die vorliegende Erfindung betrifft neue substituierte Thiazolyloxy(bzw. thio)aryl (bzw. heteraryl)derivate, Verfahren zu ihrer Herstellung und ihre Verwendung als Herbizide.

Es ist bereits bekannt, daß bestimmte Carbonsäureanilide bzw. Benzoesäurederivate herbizide Eigenschaften besitzen. (vgl. R. Wegler "Chemie der Pflanzenschutz-und Schädlingsbekämpfungsmittel" Bd. 2, Seiten 311-314 bzw, 289-295; Bd. 5 Seiten 209-217, Springer Verlag, Berlin 1970/1977, DE 3 422 007). Ihre Wirkung ist jedoch unter bestimmten Bedingungen, z.B. bei niedrigen Aufwandmengen nicht immer befriedigend. Ebenso läßt die Selektivität in manchen Fällen zu wünschen übrig.

Ferner ist bekannt, daß bestimmte Alkylheterooxyarylderivate herbizide Eigenschaften besitzen. (vgl. T. Jojima et al. Agr. Biol. Chem. 30, (9) 866 ff (1966) bzw. JA-OS 9474/1967). Die herbizide Potenz dieser Stoffe ist jedoch unter praxisrelevanten Bedingungen nicht immer ausreichend.

Es wurden nun neue Thiazolylderivate der allgemeinen Formel (I) gefunden

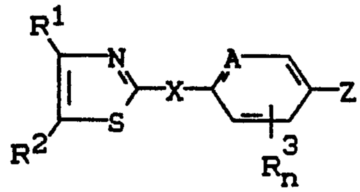

(I)

in welcher

$R^1$ für Wasserstoff oder Alkyl mit 1-6 Kohlenstoffatomen steht,

$R^2$ für Wasserstoff oder Alkyl mit 1-6 Kohlenstoffatomen steht,

mit der Maßgabe, daß $R^1$ und $R^2$ nicht gleichzeitig für Wasserstoff stehen,

A für eine (-CH=)Gruppe oder ein Stickstoffatom steht,

$R^3$ für Wasserstoff, Halogen, Cyano, Alkyl bzw. Alkoxy mit jeweils 1-6 Kohlenstoffatomen steht, wobei die Reste $R^3$ gleich oder verschieden sein können,

n für eine Zahl von 1-3 steht,

X für Sauerstoff oder Schwefel steht,

Z für den Rest

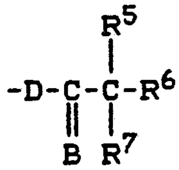 oder 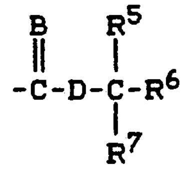

(Ia$_1$) (Ia$_2$)

oder

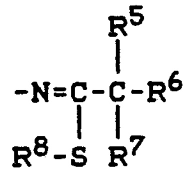 oder 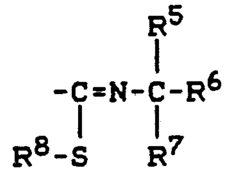

(Ib$_1$) (Ib$_2$)

oder 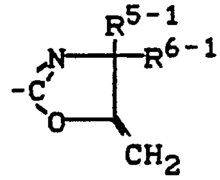

(Ic$_1$)

steht,
wobei
B für Sauerstoff oder Schwefel steht,
D für >N-$R^4$ oder Sauerstoff steht, wobei
$R^4$ für Wasserstoff, Alkyl mit 1-6 Kohlenstoffatomen oder Alkoxyalkyl mit jeweils 1 bis 6 Kohlenstoffatomen je Alkylteil, für Alkenyl oder Alkinyl mit jeweils 3 bis 6 Kohlenstoffatomen steht,
$R^5$, $R^6$ und $R^7$ jeweils unabhängig voneinander für Wasserstoff, Halogen, Cyano, Alkyl mit 1 bis 6 Kohlenstoffatomen, Halogenalkyl mit 1 bis 6 Kohlenstoffatomen und bis zu 13 Halogenatomen, Alkoxyalkyl mit jeweils 1 bis 6 Kohlenstoffatomen je Alkylteil, Alkenyl oder Alkinyl mit jeweils 2 bis 6 Kohlenstoffatomen stehen,
$R^4$ und $R^5$ gemeinsam mit dem Kohlenstoff und dem Stickstoff, an die sie gebunden sind, einen 3 bis 8 gliedrigen heterocyclischen Ring bilden, der gegebenenfalls einfach oder mehrfach, gleich oder verschieden durch Halogen, Methyl oder Ethyl substituiert ist und welcher eine oder mehrere Doppelbindungen enthalten kann,
$R^6$ und $R^7$ gemeinsam mit dem Kohlenstoffatom, an das sie gebunden sind, einen 3 bis 8 gliedrigen Ring bilden, der außer Kohlenstoff auch Sauerstoff-und/oder Schwefelatome als Ringglieder enthalten kann und der gegebenenfalls einfach oder mehrfach, gleich oder verschieden durch Halogen, Methyl und Ethyl substituiert ist und welcher eine oder mehrere Doppelbindungen enthalten kann und
$R^8$ für gegebenenfalls einfach oder mehrfach, gleich oder verschieden durch Halogen substituiertes Alkyl mit 1 bis 6 Kohlenstoffatomen, für Alkoxyalkyl mit 1 bis 6 Kohlenstoffatomen je Alkylteil, für Alkenyl oder Alkinyl mit jeweils 3 bis 6 Kohlenstoffatomen steht, und
$R^{5-1}$ und $R^{6-1}$ unabhängig voneinander jeweils für Alkyl mit 1-6 Kohlenstoffatomen stehen.

Weiterhin wurde gefunden, daß man die Thiazolylderivate der Formel (I) erhält, wenn man entweder

A) Verbindungen der allgemeinen Formel (II)

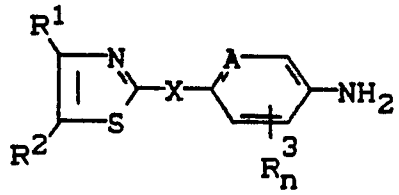

(II)

in welcher
$R^1$, $R^2$, $R^3$, n, X und A die oben angegebene Bedeutung haben, entweder

$a_1$) mit Säurehalogeniden der Formel (IIIa),

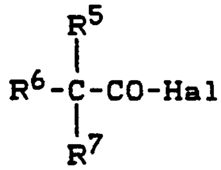

(IIIa)

in welcher
$R^5$, $R^6$, $R^7$ die oben angegebene Bedeutung haben und
Hal für Halogen steht,
gegebenenfalls in Gegenwart eines Verdünnungsmittels und gegebenenfalls in Gegenwart eines Säurebindemittels umsetzt,
oder

$a_2$) mit symmetrischen Carbonsäureanhydriden der Formel (IIIb),

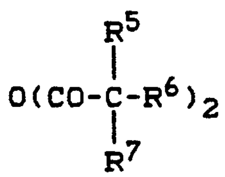

(IIIb)

in welcher
$R^5$, $R^6$ und $R^7$ die oben angegebene Bedeutung haben,
gegebenenfalls in Gegenwart eines Verdünnungsmittels umsetzt,
oder

$a_3$) mit asymmetrischen Säureanhydriden der Formel (IIIc),

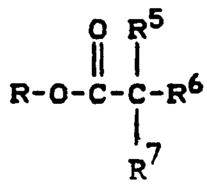

(IIIc)

in welcher
$R^5$, $R^6$ und $R^7$ die oben angegebene Bedeutung haben und
R für Alkoxycarbonyl, Alkylsulfonyl, Phenoxycarbonyl oder Phenylsulfonyl steht,
gegebenenfalls in Gegenwart eines Verdünnungsmittels umsetzt,
oder

$a_4$) mit Verbindungen der Formel (IIId),

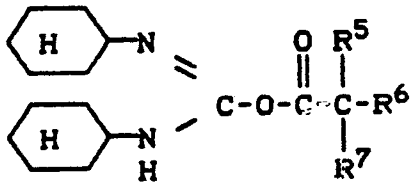

(IIId)

in welcher $R^5$, $R^6$ und $R^7$ die oben angegebene Bedeutung haben, gegebenenfalls in Gegenwart eines Verdünnungsmittels umsetzt, oder wenn man

B) Thiazolderivate der allgemeinen Formel (IV),

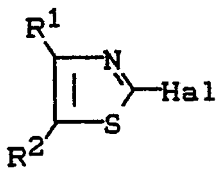

(IV)

in welcher $R^1$ und $R^2$ die oben angegebene Bedeutung haben und Hal für Halogen steht, mit Verbindungen der allgemeinen Formel (V)

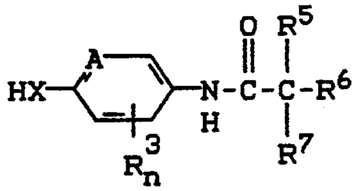

(V)

in welcher $R^3$, $R^5$, $R^6$, $R^7$, X, A und n die oben angegebene Bedeutung haben, gegebenenfalls in Gegenwart eines Verdünnungsmittels und gegebenenfalls in Gegenwart eines Säurebindemittels umsetzt, oder wenn man

C) Thiazol-Derivate der allgemeinen Formel (VI),

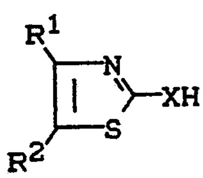

(VI)

in welcher $R^1$, $R^2$ und X die oben angegebene Bedeutung haben, entweder

$c_1$) mit 2-Halogen-pyridinderivaten der allgemeinen Formel (VIIa),

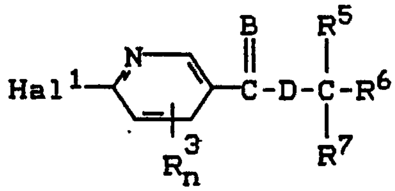

(VIIa)

in welcher
$R^3$, $R^5$, $R^6$, $R^7$, B, D und n die oben angegebene Bedeutung haben und
$Hal^1$ für Halogen steht,
gegebenenfalls in Gegenwart eines Verdünnungsmittels und gegebenenfalls in Gegenwart eines Säurebindemittels umsetzt,
oder

$c_2$) mit 6-Halogennikotinsäurederivaten der allgemeinen Formel (VIIb),

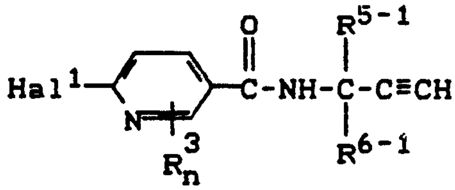

(VIIb)

in welcher
$R^3$, $R^{5-1}$, $R^{6-1}$, n und $Hal^1$ die oben angegebene Bedeutung haben
gegebenenfalls in Gegenwart eines Verdünnungsmittels und in Gegenwart einer Base umsetzt zu den Verbindungen der allgemeinen Formel ($Ic_1$)

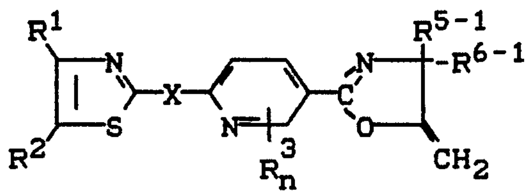

($Ic_1$)

in welcher
$R^1$, $R^2$, $R^3$, X, n, $R^{5-1}$ und $R^{6-1}$ die oben angebene Bedeutung haben,
oder wenn man

D) Verbindungen der allgemeinen Formel (VIII),

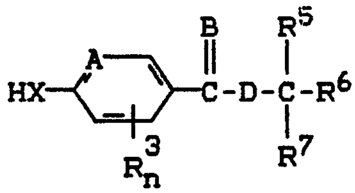

(VIII)

in welcher
$R^3$, $R^5$, $R^6$, $R^7$, A, B, D, X und n die oben angegebene Bedeutung haben,
mit Thiazol-Derivaten der allgemeinen Formel (IV),

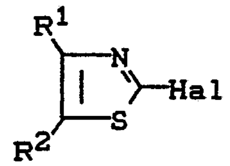

(IV)

in welcher
$R^1$ und $R^2$ die oben angegene Bedeutung haben und
Hal für Halogen steht,
gegebenenfalls in Gegenwart eines Verdünnungsmittels und gegebenenfalls in Gegenwart eines Säurebindemittels umsetzt,
oder wenn man

E) substituierte Thiazolylderivate der allgemeinen Formel ($Ia^1$)

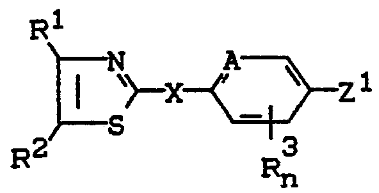

($Ia^1$)

in welcher
$Z^1$ für den Rest

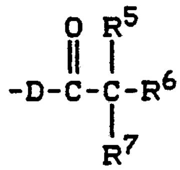

oder

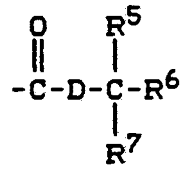

steht und
$R^1$, $R^2$, $R^3$, $R^5$, $R^6$, $R^7$, A, D, X und n die oben angegebene Bedeutung haben,
mit Schwefelungsreagenzien, wie beispielsweise Phosphor-V-sulfid oder 2,4-Bis-(4-methoxy-phenyl)-2,4-dithiono-1,2,3,4-dithia-phosphetan (Lawesson-Reagenz) umsetzt zu Verbindungen der allgemeinen Formel ($Ia^2$)

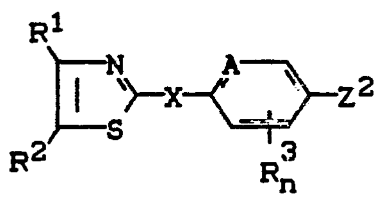

($Ia^2$)

in welcher
$Z^2$ für den Rest

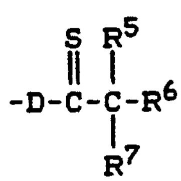

oder

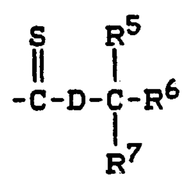

steht und
$R^1$, $R^2$, $R^3$, $R^5$, $R^6$, $R^7$, A, D, X und n die oben angegebene Bedeutung haben,
oder wenn man

F) die nach dem Verfahren (E) hergestellten Verbindungen der allgemeinen Formel ($Ia^2$), in denen D für >NH steht, mit deprotonierenden Basen und Verbindungen der allgemeinen Formel (IX),

E-$R^8$ (IX)

in welcher
$R^8$ die oben angegebene Bedeutung hat und
E für Halogen, Alkylsulfonyloxy oder Phenylsulfonyloxy steht,
gegebenenfalls in Gegenwart eines Verdünnungsmittels zu Verbindungen der allgemeinen Formeln ($Ib_1$) oder ($Ib_2$) umsetzt

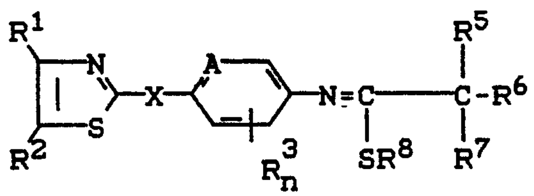

($Ib_1$)

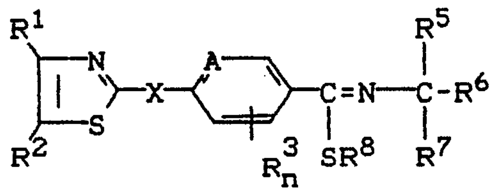

($Ib_2$)

in denen
A, $R^1$, $R^2$, $R^3$, $R^5$, $R^6$, $R^7$, $R^8$, X und n die oben angegebene Bedeutung haben
oder wenn man

G) Verbindungen der allgemeinen Formel ($Ia^3$)

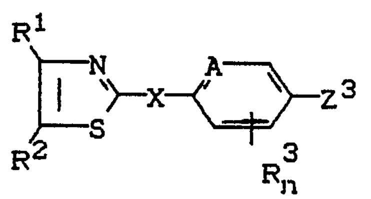

($Ia^3$)

in welcher
$R^1$, $R^2$, $R^3$, X, A und n die oben angegebene Bedeutung haben und $Z^3$ für den Rest

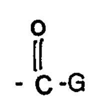

und G für Halogen, für den Rest

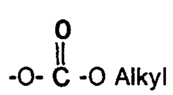

oder -O-$SO_2$-Aryl oder Imidazolyl steht, mit Verbindungen der allgemeinen Formel (XVI)

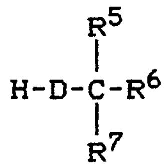

(XVI)

in welcher

D, $R^5$, $R^6$ und $R^7$ die oben angegebene Bedeutung haben gegebenenfalls in Gegenwart eines Verdünnungsmittels und gegebenenfalls in Gegenwart eines Säurebindemittels umsetzt. Schließlich wurde gefunden, daß die neuen substituierten Thiazolylderivate der allgemeinen Formel (I) sich durch hervorragende herbizide Wirkung auszeichnen.

Überraschenderweise besitzen die erfindungsgemäßen substituierten Thiazolylether bzw. -thioether der allgemeinen Formel (I) wesentlich bessere herbizide Eigenschaften als die konstitutionell ähnlichsten vorbekannten Stoffe.

Die erfindungsgemäßen Thiazolylether-und -thioetherderivate sind durch die Formel (I) allgemein definiert. Von den erfindungsgemäßen Verbindungen der allgemeinen Formel (I) sind diejenigen bevorzugt, in denen

$R^1$ für Wasserstoff oder Alkyl mit 1-4 Kohlenstoffatomen steht und

$R^2$ für Wasserstoff oder Alkyl mit 1-4 Kohlenstoffatomen steht,

mit der Maßgabe, daß $R^1$ und $R^2$ nicht gleichzeitig für Wässerstoff stehen,

A fur eine (-CH=)Gruppe oder ein Stickstoffatom steht,

$R^3$ für Wasserstoff, Fluor, Chlor, Brom, Iod, Cyano für Alkyl oder Alkoxy mit jeweils 1-4 Kohlenstoffatomen steht, wobei die Reste $R^3$ gleich oder verschieden sein können,

n für eine Zahl von 1 bis 3 steht,

X für Sauerstoff oder Schwefel steht

Z für den Rest

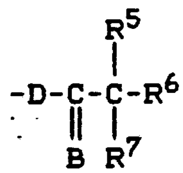

oder

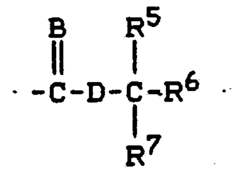

($Ia_1$) ($Ia_2$)

oder

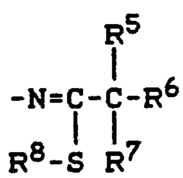

oder

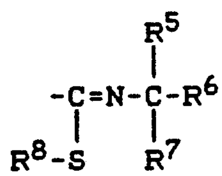

($Ib_1$) ($Ib_2$)

oder

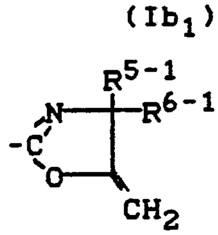

($Ic_1$)

steht,

wobei

B für Sauerstoff oder Schwefel steht,

D für >N-$R^4$ oder Sauerstoff steht, wobei

$R^4$ für Wasserstoff, Alkyl mit 1-4 Kohlenstoffatomen oder Alkoxyalkyl mit jeweils 1 bis 4 Kohlenstoffatomen je Alkylteil, für Alkenyl oder Alkinyl mit jeweils 3 bis 4 Kohlenstoffatomen steht,

$R^5$, $R^6$ und $R^7$ jeweils unabhängig voneinander für Wasserstoff, Fluor, Chlor, Brom, Iod, Cyano, Alkyl mit 1 bis 5 Kohlenstoffatomen, Halogenalkyl mit 1 bis 4 Kohlenstoffatomen und 1 bis 9 Halogenatomen, Alkoxyalkyl mit jeweils 1 bis 4 Kohlenstoffatomen je Alkylteil, Alkenyl oder Alkinyl mit jeweils 2 bis 4 Kohlenstoffatomen stehen,

$R^4$ und $R^5$ gemeinsam mit dem Kohlenstoff und dem Stickstoff, an die sie gebunden sind, einen 3 bis 7 gliedrigen heterocyclischen Ring bilden, der gegebenenfalls einfach bis zehnfach, gleich oder verschieden durch Fluor, Chlor, Brom, Methyl oder Ethyl substituiert ist und welcher eine bis zwei Doppelbindungen enthalten kann,

$R^6$ und $R^7$ gemeinsam mit dem Kohlenstoffatom, an das sie gebunden sind, einen 3 bis 7 gliedrigen Ring bilden, der außer Kohlenstoff auch Sauerstoff-und/oder Schwefelatome als Ringglieder enthalten kann und der gegebenenfalls einfach bis zwölffach, gleich oder verschieden durch Fluor, Chlor, Brom, Methyl oder Ethyl substituiert ist und welcher eine bis drei Doppelbindungen enthalten kann und

$R^8$ für gegebenenfalls einfach bis fünffach, gleich oder verschieden durch Fluor, Chlor oder Brom substituiertes Alkyl mit 1 bis 5 Kohlenstoffatomen, für Alkoxyalkyl mit 1 bis 5 Kohlenstoffatomen je Alkylteil, für Alkenyl oder Alkinyl mit jeweils 3 bis 4 Kohlenstoffatomen steht, und

$R^{5-1}$ und $R^{6-1}$ unabhängig voneinander jeweils für Alkyl mit 1 bis 4 Kohlenstoffatomen stehen.

Als besonders bevorzugt sind die erfindungsgemäßen Thiazolylderivate der allgemeinen Formel (I) zu erwähnen, in denen

$R^1$ für Wasserstoff, Methyl oder Ethyl steht und

$R^2$ für Wasserstoff oder Alkyl mit 1-3 Kohlenstoffatomen steht, mit der Maßgabe, daß $R^1$ und $R^2$ nicht gleichzeitig für Wasserstoff stehen,

A für eine (-CH=)Gruppe oder ein Stickstoffatom steht,

$R^3$ für Wasserstoff, Fluor, Chlor, Cyano, Methyl, Ethyl, Methoxy oder Ethoxy steht, wobei die Reste $R^3$ gleich oder verschieden sein können,

n für eine Zahl von 1 bis 3 steht,

X für Sauerstoff oder Schwefel steht

Z für den Rest

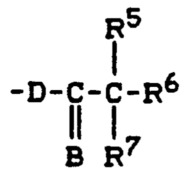

$(Ia_1)$

oder

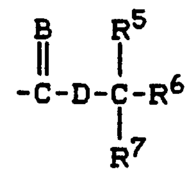

$(Ia_2)$

oder

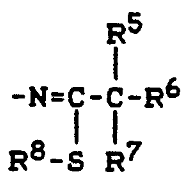

$(Ib_1)$

oder

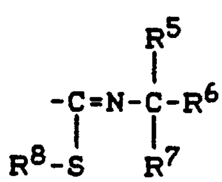

$(Ib_2)$

oder

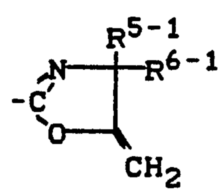

$(Ic_1)$

steht,
wobei
B für Sauerstoff oder Schwefel steht,
D für >N-$R^4$ oder Sauerstoff steht, wobei
$R^4$ für Wasserstoff, Methyl, Ethyl, Methoxymethyl, Methoxyethyl, Ethoxymethyl, Ethoxyethyl, Allyl oder Propargyl steht,
$R^5$ und $R^6$ unabhängig voneinander für Wasserstoff, Fluor, Chlor, Brom, Methyl, Ethyl, n-Propyl, iso-Propyl, Halogen-$C_1$-$C_4$-alkyl mit 1 bis 5 gleichen oder verschiedenen Fluor-und Chloratomen steht,
$R^7$ für Wasserstoff, Fluor, Chlor, Brom, Cyano, Methyl, Ethyl, n-Propyl, iso-Propyl, n-Butyl, iso-Butyl, sec.-Butyl, tert.-Butyl, n-Pentyl, iso-Pentyl, iso-Pentyl, neo-Pentyl, tert.-Pentyl, Alkoxyalkyl mit jeweils 1 bis 3 Kohlenstoffatomen je Alkylteil, Vinyl, Allyl, Ethinyl, Propargyl, Halogen-$C_1$-$C_4$-alkyl mit 1 bis 5 gleichen oder verschiedenen Fluor-und Chloratomen steht oder
$R^4$ und $R^5$ gemeinsam mit dem Kohlenstoff und dem Stickstoff, an die sie gebunden sind, einen 3 bis 6 gliedrigen heterocyclischen Ring bilden, der gegebenenfalls einfach bis achtfach, gleich oder verschieden durch Fluor, Chlor, Methyl und Ethyl substituiert ist und welcher eine oder zwei Doppelbindungen enthalten kann, oder
$R^6$ und $R^7$ gemeinsam mit dem Kohlenstoff, an den sie gebunden sind, einen 3 bis 6 gliedrigen Ring bilden, der außer Kohlenstoff auch Sauerstoff und/oder Schwefel als Ringglieder enthalten kann und der gegebenenfalls einfach bis zehnfach, gleich oder verschieden durch Fluor, Chlor, Methyl oder Ethyl substituiert ist und der eine bis zwei Doppelbindungen enthalten kann, und
$R^8$ für gegebenenfalls einfach bis dreifach, gleich oder verschieden durch Fluor, Chlor und Brom substituiertes $C_1$-$C_2$-Alkyl, Methoxymethyl, Methoxyethyl, Ethoxymethyl, Ethoxyethyl, Allyl oder Propargyl steht und
$R^{5-1}$ und $R^{6-1}$ unabhängig voneinander für Methyl, Ethyl oder iso-Propyl stehen.

Verwendet man gemäß Verfahren A (Variante $a_1$) 4-(4,5-Dimethylthiazolyl-2-thio)anilin und Pivalsäurechlorid als Ausgangsstoffe, so kann der Verlauf des erfindungsgemäßen Verfahrens durch das folgende Formelschema wiedergegeben werden:

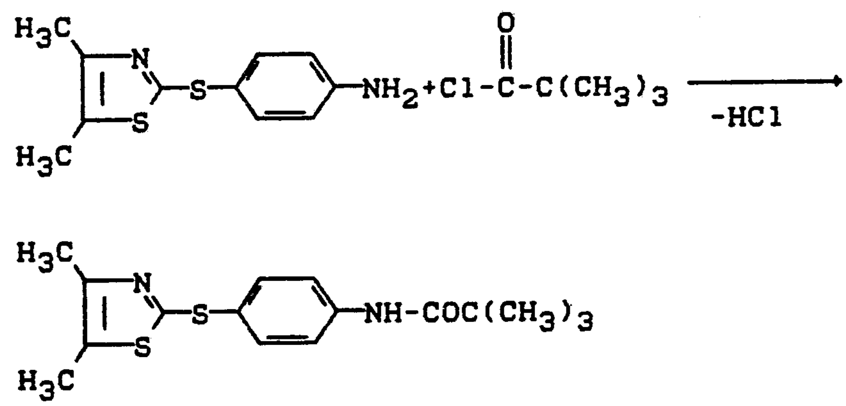


Verwendet man gemäß Verfahren A (Variante $a_2$) 4-(4,5-Dimethylthiazolyl-2-thio)anilin und Isobuttersäureanhydrid als Ausgangsstoffe, so kann der Verlauf des erfindungsgemäßen Verfahrens durch folgendes Formelschema wiedergegeben werden:

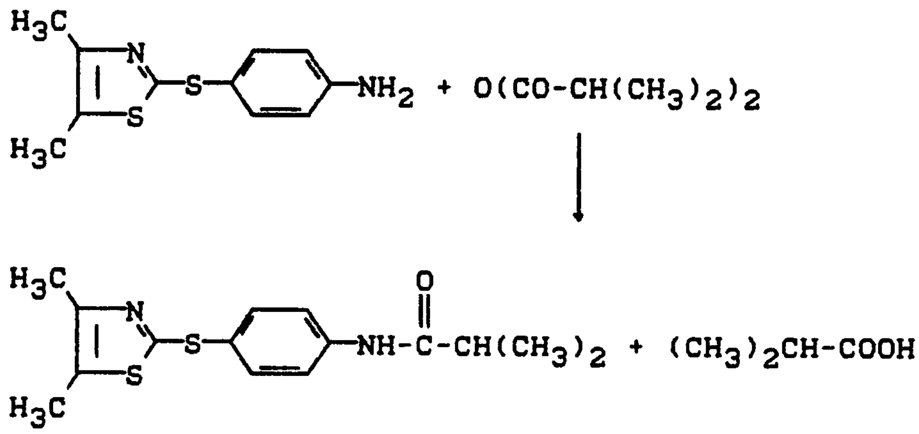

Verwendet man 4-(4,5-Dimethyl-thiazolyl-2-thio)-anilin und Pivaloylkohlensäure-ethylesteranhydrid als Ausgangsstoffe, so kann der Verlauf der erfindungsgemäßen Verfahrens A (Variante $a_3$) durch das folgende Formelschema wiedergegeben werden:

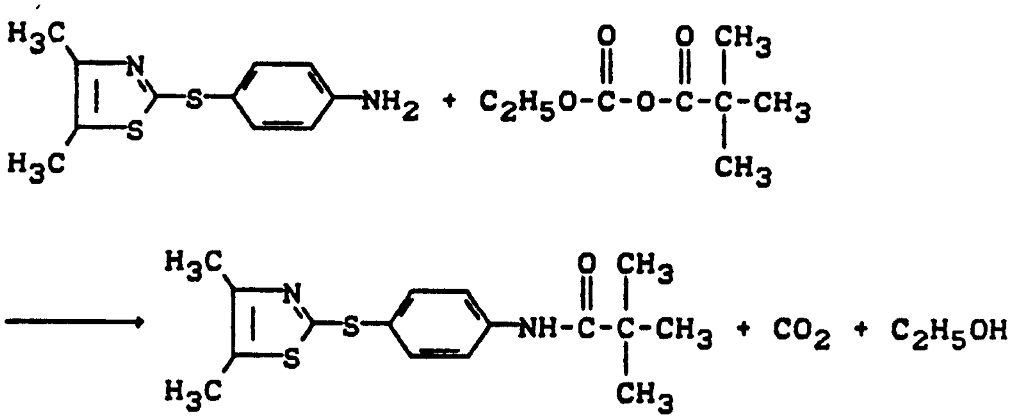

Verwendet man 4-(4,5-Dimethyl-thiazolyl-2-thio)-anilin und O-Pivaloyl-dicyclohexyl-isoharnstoff als Ausgangsstoffe, so kann der Verlauf des erfindungsgemäßen Verfahrens A (Variante $a_4$) durch das folgende Formelschema wiedergegeben werden:

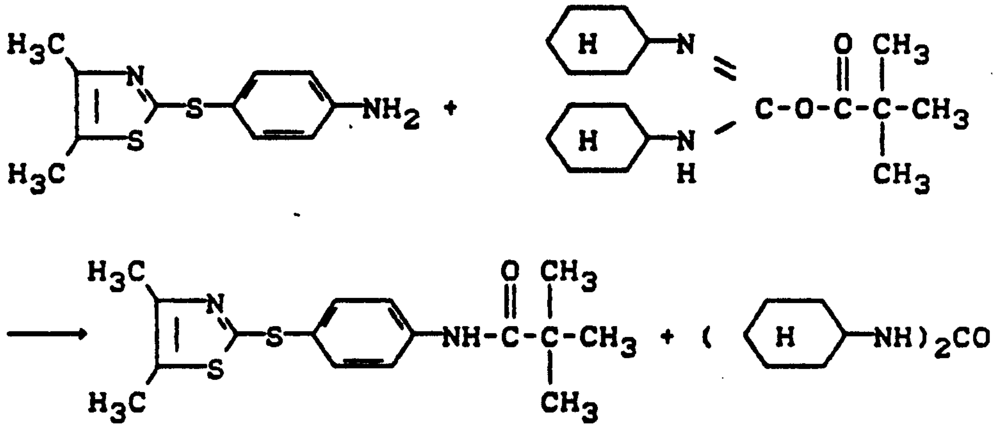

Verwendet man 2-Chlor-4,5-dimethylthiazol und 4-Pivaloylamino-phenol als Ausgangsstoffe, so kann der Verlauf der erfindungsgemäßen Verfahrens B durch das folgende Formelschema wiedergegeben werden:

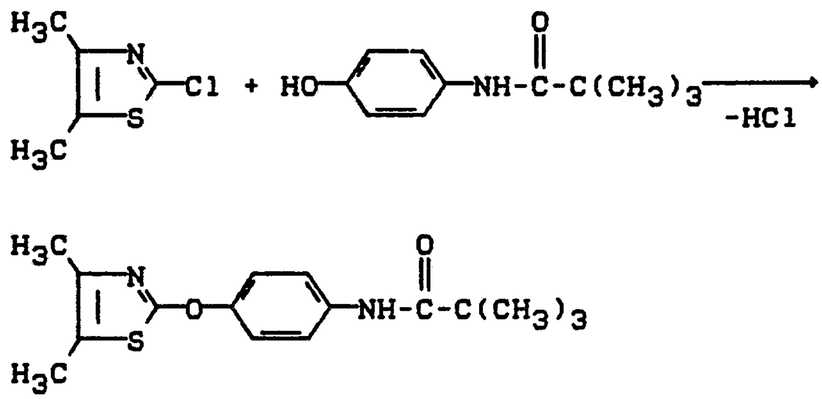


Verwendet man 2-Mercapto-4,5-dimethylthiazol und 6-Chlornikotinsäure-t-butylamid als Ausgangsstoffe, so kann der Verlauf des erfindungsgemäßen Verfahrens C (Variante $C_1$) durch das folgende Formelschema wiedergegeben werden:

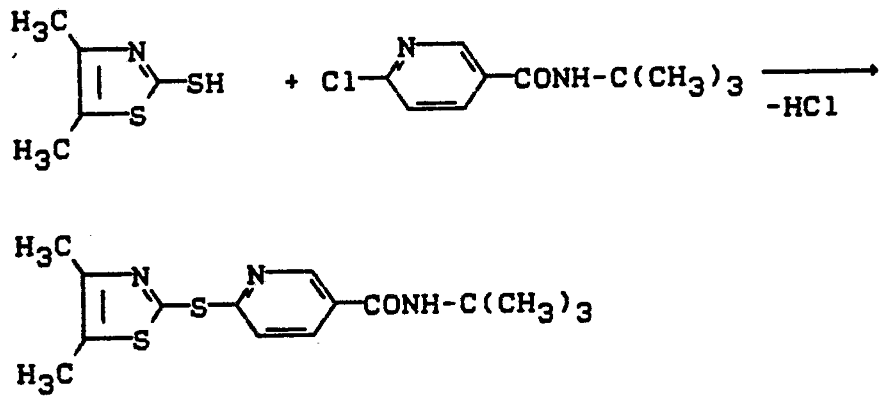


Verwendet man gemäß Verfahren C (Variante $C_2$) 2-Mercapto-4,5-dimethylthiazol und 6-Chlornicotinsäuredimethylpropargylamid als Ausgangsmaterialien, so läßt sich der Reaktionsverlauf durch folgendes Schema darstellen:

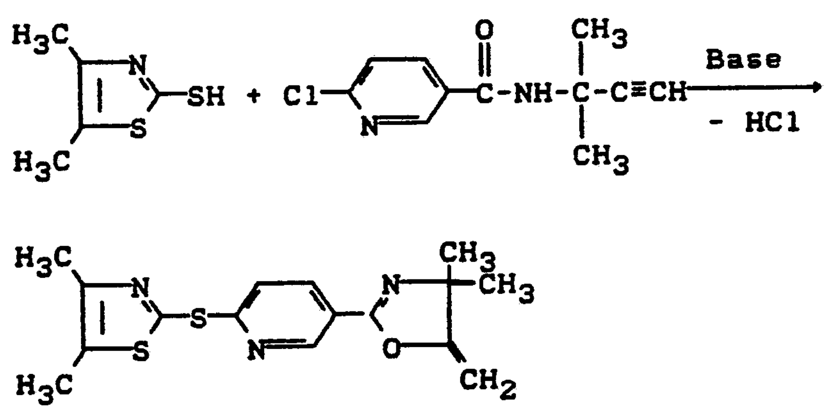


Verwendet man gemäß Verfahren D 4-Mercaptobenzoesäure(t)-butylamid und 2-Chlor-4,5-dimethylthiazol als Ausgangsmaterialien so wird der Reaktionsverlauf durch folgendes Schema beschrieben:

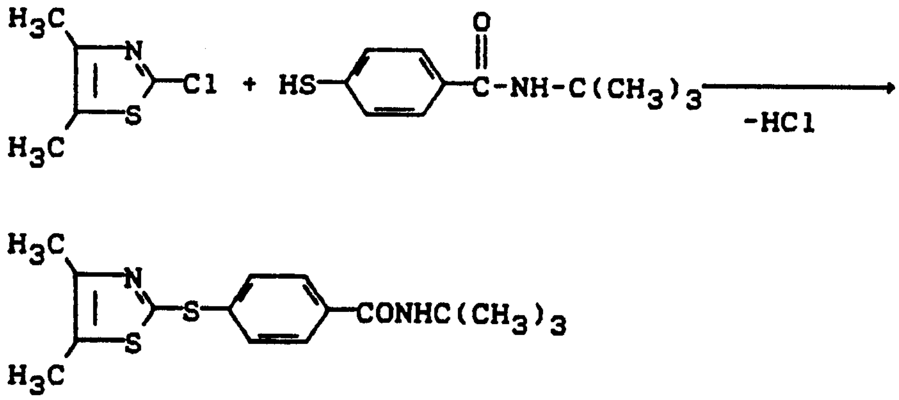


Verwendet man gemäß Verfahren E 2-(4-t-Butylaminocarbonylphenyl-thio)-4,5-dimethylthiazol und Lawesson-Reagenz als Reaktionspartner so wird der Reaktionsverlauf durch das folgende Schema beschrieben:

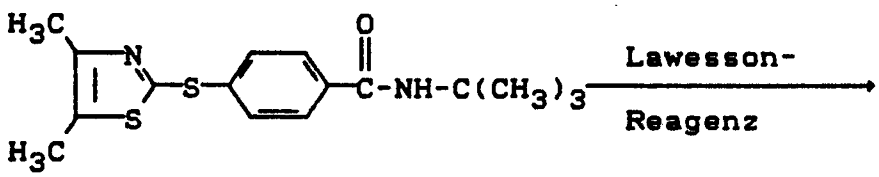


Verwendet man gemäß Verfahren F 2-(4-t-Butylamino-thiocarbonylphenyl-thio)-4,5-dimethylthiazol und Methyliodid als Ausgangsstoffe, so wird der Reaktionsverlauf durch folgendes allgemeine Formelschema beschrieben:

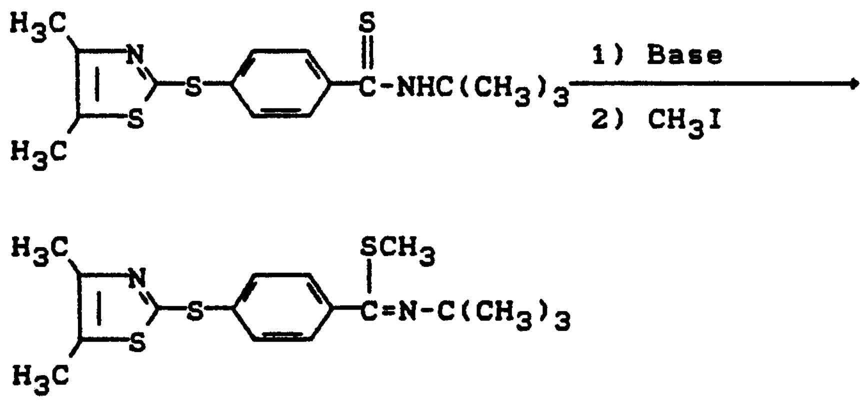


Verwendet man gemäß Verfahren G 4-(4,5-Dimethylthiazolyl-2-thio)benzoesäurechlorid und t-Butylamin als Ausgangsstoffe, so wird das erfindungsgemäße Verfahren durch das folgende Schema beschrieben:

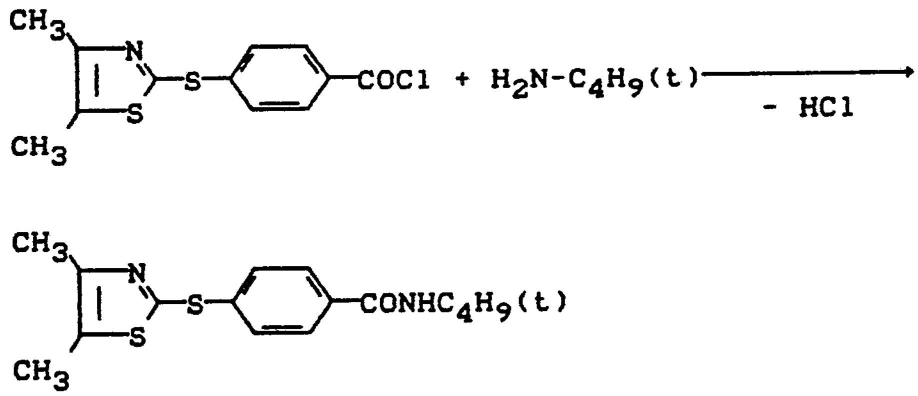


Die bei dem erfindungsgemäßen Verfahren (A) als Ausgangsstoffe benötigten Anilin-Derivate sind durch die Formel (II) allgemein definiert. In dieser Formel haben $R^1$, $R^2$ $R^3$, A, X und n vorzugsweise diejenigen Bedeutungen, die bereits im Zusammenhang mit der Beschreibung der erfindungsgemäßen Stoffe der allgemeinen Formel (I) vorzugsweise für diese Reste bzw. diesen Index genannt wurden.

Die Anilin-Derivate der allgemeinen Formel (II) sind teilweise bekannt. Sie lassen sich herstellen, indem man

α) Halogenthiazole der Formel

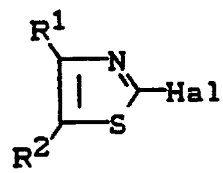

(IV)

in welcher
$R^1$ und $R^2$ und Hal die oben angegebene Bedeutung haben
mit Verbindungen der allgemeinen Formel (X)

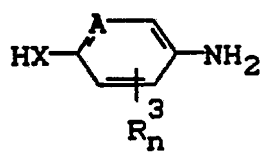

(X)

in welcher
A, $R^3$, X und n die oben angegebene Bedeutung haben,
in Gegenwart eines Säurebindemittels sowie gegebenenfalls in Gegenwart eines Verdünnungsmittels umsetzt,
oder

β) Thiazol-Derivate der allgemeinen Formel (XI)

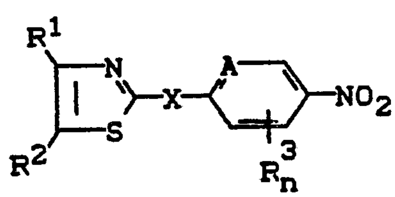

(XI)

in welcher
A, $R^1$, $R^2$, $R^3$, X und n die oben angegebene Bedeutung haben,
gegebenenfalls in Gegenwart eines Verdünnungsmittels nach üblichen Methoden reduziert.

Die bei dem obigen Verfahren (Variante α) als Ausgangsstoffe benötigten Thiazol-Derivate sind durch die allgemeine Formel (IV) definiert. In dieser Formel haben $R^1$ und $R^2$ vorzugsweise diejenigen Bedeutun-

gen, die bereits im Zusammenhang mit der Beschreibung der erfindungsgemäßen Stoffe der Formel (I) vorzugsweise bzw. besonders bevorzugt für diesen Rest genannt wurden. Hal steht vorzugsweise für Fluor, Chlor oder Brom.

Als Beispiele für Thiazolderivate der allgemeinen Formel (IV) seien genannt:

2-Chlor-4,5-dimethylthiazol
2-Chlor-5-methylthiazol
2-Chlor-4-methylthiazol
2-Chlor-4-ethylthiazol
2-Chlor-4-propylthiazol
2-Chlor-4-i-propylthiazol
2-Chlor-5-ethylthiazol
2-Chlor-5-n-propylthiazol
2-Chlor-5-i-propylthiazol
2-Chlor-5-n-butylthiazol
2-Chlor-5-i-butylthiazol
2-Chlor-5-t-butylthiazol
2-Chlor-4-ethyl-5-methylthiazol
2-Chlor-4-methyl-5-ethylthiazol.

Die Thiazolderivate der allgemeinen Formel (IV) sind bekannt oder lassen sich nach im Prinzip bekannten Methoden in einfacher Weise herstellen (siehe hierzu: Thiazole and its derivatives Bd. I, 565 ff. in: A. Weißberger, The Chemistry of heterocyclic compounds, John Wiley and Sons, 1979).

Die bei dem Verfahren (α) weiterhin als Ausgangsstoffe benötigten Aminoverbindungen sind durch die allgemeine Formel (X) definiert. In dieser Formel haben A, $R^3$, X und n vorzugsweise bzw. besonders bevorzugt diejenigen Bedeutungen, die bereits im Zusammenhang mit der Beschreibung der erfindungsgemäßen Stoffe der allgemeinen Formel (I) vorzugsweise für diese Reste bzw. für diesen Index genannt wurden.

Als Beispiele für diese Aminoverbindungen der allgemeinen Formel (X) seien genannt:

4-Amino-phenol
2-Chlor-4-amino-phenol
3-Chlor-4-amino-phenol
2,5-Dichlor-4-amino-phenol
2,6-Dichlor-4-amino-phenol
2-Methyl-4-amino-phenol
3-Methyl-4-amino-phenol
2-Chlor-5-methyl-4-amino-phenol
4-Amino-thiophenol
2-Chlor-4-amino-thiophenol
3-Chlor-4-amino-thiophenol
2-Methyl-4-amino-thiophenol
3-Methyl-4-amino-thiophenol
2-Mercapto-5-amino-pyridin

Die 4-Aminoderivate der allgemeinen Formel (X) sind bekannt oder lassen sich nach im Prinzip bekannten Methoden in einfacher Weise herstellen.

Als Säurebindemittel können bei der Durchführung des Verfahrens (α) alle üblicherweise für derartige Umsetzungen verwendbaren Säureakzeptoren verwendet werden. Vorzugsweise verwendbar sind Alkali-und Erdalkalioxide, -hydroxide und -carbonate, wie Natriumhydroxid, Kaliumhydroxid, Calciumoxid, Natriumcarbonat und Kaliumcarbonat, ferner Alkali-alkoholate, -amide und -hydride, wie zum Beispiel Natriummethylat, Natrium-ethylat, Kalium-tert.-butylat, Natrium-amid und Natrium-hydrid.

Als Verdünnungsmittel können bei der Durchführung des Verfahrens (α) alle üblichen inerten organischen Solventien verwendet werden. Vorzugsweise in Frage kommen Kohlenwasserstoffe, wie Benzin, Toluol und Xylol, ferner Ether, wie Dioxan, Glykoldimethylether und Diglykoldimethylether, außerdem Nitrile, wie Acetonitril, und auch stark polare Solventien, wie Dimethylsulfoxid, Sulfolan und Dimethylformamid.

Die Reaktionstemperaturen können bei der Durchführung des Verfahrens (α) innerhalb eines größeren Bereiches variiert werden. Im allgemeinen arbeitet man bei Temperaturen zwischen 0°C und 200°C, vorzugsweise zwischen 50°C und 150°C.

Die Umsetzung nach dem Verfahren (α) wird im allgemeinen unter Normaldruck vorgenommen.

Bei der Durchführung des Verfahrens (α) setzt man die Ausgangsstoffe der allgemeinen Formeln (IV)

und (X) im allgemeinen in angenähert äquimolaren Mengen um. Es ist jedoch auch möglich, die eine oder andere Komponente in einem größeren Überschuß zu verwenden. Die Aufarbeitung erfolgt nach üblichen Methoden.

Die bei dem obigen Verfahren (Variante $\beta$) als Ausgangsstoffe benutzten Nitroverbindungen sind durch die allgemeine Formel (XI) definiert. In dieser Formel haben die Reste $R^1$, $R^2$, $R^3$, A, X und n vorzugsweise diejenige Bedeutung, die bereits im Zusammenhang mit der Beschreibung der erfindungsgemäßen Stoffe der Formel (I) vorzugsweise für diese Reste genannt wurden.

Die Verbindungen der allgemeinen Formel (XI) sind bekannt oder lassen sich nach im Prinzip bekannten Methoden in einfacher Weise herstellen. So erhält man Verbindungen der allgemeinen Formel (XI) zum Beispiel dadurch, daß man Thiazolderivate der allgemeinen Formel (IV)

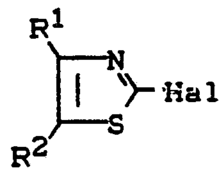

(IV)

in welcher
$R^1$, $R^2$ und Hal die oben angegebene Bedeutung haben,
mit den Nitroderivaten der allgemeinen Formel (XII)

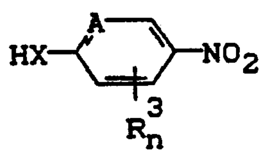

(XII)

in welcher
A, $R^3$, n und X die oben angegebene Bedeutung haben,
in Gegenwart eines Säurebindemittels und gegebenenfalls in Gegenwart eines Verdünnungsmittels bei Temperaturen zwischen 0°C und 200°C, vorzugsweise zwischen 50°C und 150°C, umsetzt. Als Säurebindemittel und Verdünnungsmittel kommen hierbei vorzugsweise diejenigen Stoffe in Betracht, die bereits im Zusammenhang mit dem Verfahren ($\alpha$) als vorzugsweise verwendbare Säureakzeptoren und Solventien genannt wurden.

Als Reduktionsmittel kommen bei dem Verfahren ($\beta$) alle diejenigen Stoffe in Frage, die üblicherweise zur Reduktion aromatischer Nitroverbindungen eingesetzt werden. Vorzugsweise verwendbar sind elementare Metalle, wie Eisen, Zink und Zinn, ferner Metallverbindungen in niederen Wertigkeitsstufen, wie Eisen (II)-und Zinn (II)-Salze, und außerdem Nichtmetall-Verbindungen in niederen Wertigkeitsstufen, wie zum Beispiel Salze des Schwefelwasserstoffes, Alkalisulfite und Alkalidithionite. Im übrigen kann die Reduktion auch durch katalytische Hydrierung mit Wasserstoff in Gegenwart eines Katalysators, wie zum Beispiel Raney-Nickel, erfolgen.

Als Verdünnungsmittel kommen bei dem Verfahren ($\beta$) alle üblichen für derartige Reduktionen geeigneten organischen Solvantien in Betracht. Die Reaktionstemperaturen können innerhalb eines größeren Bereiches variiert werden. Sie entsprechen den Temperaturen, die bei analogen Reaktionen angewandt werden. Ebenso kann die Reaktion bei den Drucken durchgeführt werden, die bei analogen Reaktionen angewandt werden.

Die bei dem erfindungsgemäßen Verfahren A (Variante $a_1$) als Reaktionskomponenten benötigten Säurehalogenide sind durch die allgemeine Formel (III a) eindeutig definiert. In dieser Formel haben $R^5$, $R^6$ und $R^7$ vorzugsweise diejenigen Bedeutungen, die bereits im Zusammenhang mit der Beschreibung der erfindungsgemäßen Stoffe der allgemeinen Formel (I) vorzugsweise für diese Reste genannt wurden. Hal steht vorzugsweise für Fluor, Chlor und Brom.

Die Säurehalogenide der allgemeinen Formel (IIIa) sind bekannt oder lassen sich nach im Prinzip bekannten Methoden in einfacher Weise herstellen.

Als Säurebindemittel kommen bei der Umsetzung nach dem erfindungsgemäßen Verfahren A (Variante $a_1$) alle üblichen Säureakzeptoren in Betracht. Vorzugsweise verwendbar sind tertiäre Amine, wie Triethylamin, Pyridin und N,N-Dimethyl-anilin, ferner Erdalkalimetalloxide; geeignet sind Carbonate z.B. Natriumcarbonat, Kaliumcarbonat und Calciumcarbonat. Es ist auch möglich, die jeweiligen Verbindungen der

allgemeinen Formel (II) gleichzeitig als Säurebindemittel zu verwenden. Dazu muß die betreffende Verbindung dann zumindest in solcher Menge eingesetzt werden, daß der freiwerdende Halogenwasserstoff gebunden werden kann.

Als Verdünnungsmittel können bei dem erfindungsgemäßen Verfahren A (Variante $a_1$) alle gegenüber Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methyl-iso propylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Die Reaktionstemperaturen können bei der Durchführung des erfindungsgemäßen Verfahrens A (Variante $a_1$) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren A (Variante $a_1$) wird im allgemeinen unter Normaldruck durchgeführt.

Bei der Durchführung des erfindungsgemäßen Verfahrens A (Variante $a_1$) werden die Ausgangsstoffe der allgemeinen Formeln (II) und (IIIa) im allgemeinen in angenähert äquivalenten Mengen verwendt. Es ist jedoch auch möglich, die eine oder andere Komponente in einem größeren Überschuß einzusetzen. Die Aufarbeitung erfolgt danach nach üblichen Methoden. Im allgemeinen geht man so vor, daß man ausgefallene Salze entfernt und das verbleibende Reaktionsgemisch durch Abziehen des Verdünnungsmittels einengt. Arbeitet man in Gegenwart von Wasser oder von mit Wasser mischbaren Solventien, so kann man auch so verfahren, daß man das Reaktionsgemisch mit Wasser verdünnt, das entstehende Gemisch absaugt oder mit einem Wasser wenig mischbaren organischen Lösungsmittel extrahiert, die organische Phase wäscht, einengt und den verbleibenden Rückstand gegebenenfalls üblichen Reinigungsverfahren unterwirft.

Die bei dem erfindungsgemäßen Verfahren A (Variante $a_2$) als Reaktionskomponenten benötigten symmetrischen Carbonsäure-anhydride sind durch die allgemeine Formel (IIIb) eindeutig definiert. In dieser Formel haben $R^5$, $R^6$ und $R^7$ vorzugsweise diejenigen Bedeutungen, die bereits im Zusammenhang mit der Beschreibung der erfindungsgemäßen Stoffe der allgemeinen Formel (I) vorzugsweise für diese Reste genannt wurden.

Die symmetrischen Carbonsäureanhydride der allgemeinen Formel (IIIb) sind bekannt oder lassen sich nach im Prinzip bekannten Methoden in einfacher Weise herstellen.

Als Verdünnungsmittel können bei der Durchführung des erfindungsgemäßen Verfahrens A (Variante $a_2$) vorzugsweise diejenigen Verdünnungsmittel verwendet werden, die auch bei dem Verfahren A (Variante $a_1$) vorzugsweise in Betracht kommen. Im übrigen kann auch ein im Überschuß eingesetztes Carbonsäureanhydrid der allgemeinen Formel (IIIb) gleichzeitig als Verdünnungsmittel fungieren.

Die Reaktionstemperaturen können auch bei dem erfindungsgemäßen Verfahren A (Variante $a_2$) innerhalb eines größeren Bereiches variiert werden. Im allgemeinen arbeitet man bei Temperaturen zwischen -20°C und +150°C, vorzugsweise zwischen 0 und 100°C.

Das erfindungsgemäße Verfahren A (Variante $a_2$) wird im allgemeinen unter Normaldruck durchgeführt.

Bei der Durchführung des erfindungsgemäßen Verfahrens A (Variante $a_2$) werden die Ausgangsstoffe der allgemeinen Formeln (II) und (IIIb) im allgemeinen in angenähert äquivalenten Mengen verwendet. Es ist jedoch auch möglich, das Carbonsäureanhydrid in einem größeren Überschuß einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Die bei dem erfindungsgemäßen Verfahren A (Variante $a_3$) als Reaktionskomponenten benötigten asymmetrischen Säureanhydride sind durch die allgemeine Formel (IIIc) eindeutig definiert. In dieser Formel haben $R^5$, $R^6$ und $R^7$ vorzugsweise diejenigen Bedeutungen, die bereits im Zusammenhang mit der Beschreibung der erfindungsgemäßen Stoffe der allgemeinen Formel (I) vorzugsweise für diese Reste genannt wurden. R steht vorzugsweise für Alkyl mit 1 oder 2 Kohlenstoffatomen oder für Phenyl.

Die asymmetrischen Säureanhydride der allgemeinen Formel (IIIc) sind bekannt oder lassen sich nach im Prinzip bekannten Methoden in einfacher Weise herstellen. So erhält man Verbindungen der allgemeinen Formel (IIIc) dadurch, daß man Carbonsäuren der Formel (IIIe)

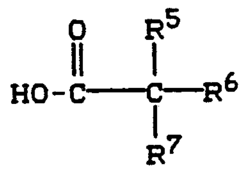

(IIIe)

in welcher
$R^5$, $R^6$ und $R^7$ die oben angegebene Bedeutung haben,
mit Kohlensäure-ester-chloriden der allgemeinen Formel (XIII)

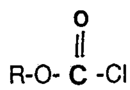

(XIII)

in welcher
R die oben angegebene Bedeutung hat,
in Gegenwart eines Verdünnungsmittels, wie z.B. Methylenchlorid, und in Gegenwart eines Säurebindemittels, wie z.B. Triethylamin, bei Temperaturen zwischen -20°C und +100°C, vorzugsweise zwischen 0 und 50°C, umsetzt. Die asymmetrischen Säureanhydride der allgemeinen Formel (IIIc) werden im allgemeinen nicht in reiner Form isoliert, sondern in der anfallenden Form, gegebenenfalls nach vorheriger Entfernung von Verdünnungsmittel und/oder Salzen, weiter verarbeitet.

Als Verdünnungsmittel können bei der Durchführung des erfindungsgemäßen Verfahrens A (Variante $a_3$) vorzugsweise diejenigen Verdünnungsmittel verwendet werden, die auch bei dem Verfahren A (Variante $a_1$) vorzugsweise in Betracht kommen. Im übrigen kann auch im Überschuß eingesetztes Säureanhydrid der allgemeinen Formel (IIIc) gleichzeitig als Verdünnungsmittel fungieren.

Die Reaktionstemperaturen können auch bei dem erfindungsgemäßen Verfahren A (Variante $a_3$) innerhalb eines größeren Bereiches variiert werden. Im allgemeinen arbeitet man bei Temperaturen zwischen -20°C und +150°C, vorzugsweise zwischen 0°C und 100°C.

Das erfindungsgemäße Verfahren (A-$a_3$) wird im allgemeinen unter Normaldruck durchgeführt.

Bei der Durchführung des erfindungsgemäßen Verfahrens A (Variante $a_3$) werden die Ausgangsstoffe der allgemeinen Formeln (II) und (IIIc) im allgemeinen in angenähert äquivalenten Mengen verwendet. Es ist jedoch auch möglich, das Säureanhydrid in einem größeren Überschuß einzusetzen. Die Aufarbeitung erfolgt nach üblichen Methoden.

Die bei dem erfindungsgemäßen Verfahren A (Variante $a_4$) als Reaktionskomponenten benötigten Verbindungen sind durch die allgemeine Formel (IIId) eindeutig definiert. In dieser Formel haben $R^5$, $R^6$ und $R^7$ vorzugsweise diejenigen Bedeutungen, die bereits im Zusammenhang mit der Beschreibung der erfindungsgemäßen Stoffe der allgemeinen Formel (I) vorzugsweise für diese Reste genannt wurden.

Die Verbindungen der allgemeinen Formel (IIId) sind bekannt oder lassen sich nach im Prinzip bekannten Verfahren in einfacher Weise herstellen. So erhält man Verbindungen der allgemeinen Formel (IIId) dadurch, daß man Carbonsäuren der allgemeinen Formel

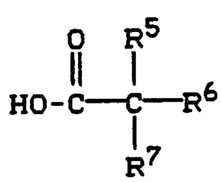

(IIIe)

in welcher
$R^5$, $R^6$ und $R^7$ die oben angegebene Bedeutung haben,
mit Dicyclohexylcarbodiimid der Formel (XIV)

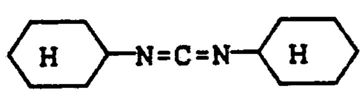

(XIV)

umsetzt.

Als Verdünnungsmittel können bei der Durchführung des erfindungsgemäßen Verfahrens A (Variante

$a_4$) vorzugsweise diejenigen Verdünnungsmittel verwendet werden, die auch bei dem Verfahren A (Variante $a_1$) vorzugsweise in Betracht kommen.

Die Reaktionstemperaturen können auch bei dem erfindungsgemäßen Verfahren A (Variante $a_4$) innerhalb eines größeren Bereiches variiert werden. Im allgemeinen arbeitet man bei Temperaturen zwischen -20°C und +150°C, vorzugsweise zwischen 0 und 100°C.

Das erfindungsgemäße Verfahren A (Variante $a_4$) wird im allgemeinen unter Normaldruck durchgeführt.

Bei der Durchführung des erfindungsgemäßen Verfahrens A (Variante $a_4$) werden die Ausgangsstoffe der allgemeinen Formeln (II) und (IIId) im allgemeinen in angenähert äquivalenten Mengen verwendet. Die Aufarbeitung erfolgt nach üblichen Methoden.

Die bei dem erfindungsgemäßen Verfahren (B) als Ausgangsstoffe benötigten Halogenthiazole der allgemeinen Formel (IV) wurden bereits im Zusammenhang mit der Beschreibung des Verfahrens A abgehandelt.

Die bei dem erfindungsgemäßen Verfahren (B) weiterhin als Ausgangsstoffe benötigten Acylamino-Derivate sind durch die allgemeine Formel (V) eindeutig definiert. In dieser Formel haben $R^5$, $R^6$, $R^7$, A, X, $R^3$ und n vorzugsweise diejenigen Bedeutungen, die bereits im Zusammenhang mit der Beschreibung der Stoffe der allgemeinen Formel (I) vorzugsweise für diese Reste bzw. für diesen Index genannt wurden.

Die Verbindungen der allgemeinen Formel (V) sind bekannt oder lassen sich nach im Prinzip bekannten Methoden herstellen. So erhält man Acylamino-Derivate der allgemeinen Formel (V) z.B. dadurch, daß man Aminoderivate der allgemeinen Formel

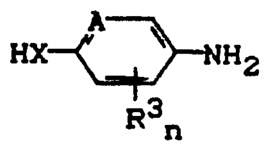

(X)

in welcher

A, X, $R^3$ und n die oben angegebene Bedeutung haben,

mit Säurehalogeniden der allgemeinen Formel (IIIa)

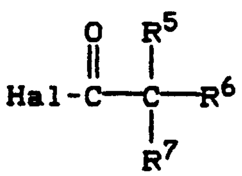

(IIIa)

in welcher

$R^5$, $R^6$, $R^7$ und Hal die oben angegebene Bedeutung haben,

gegebenenfalls in Gegenwart eines Verdünnungsmittels und gegebenenfalls in Gegenwart eines Säurebindemittels umsetzt. Die Reaktionsbedingungen entsprechen dabei denjenigen, die auch bei der Durchführung des Verfahrens A (Variante $a_1$) angewandt werden.

Als Säurebindemittel können bei der Durchführung des erfindungsgemäßen Verfahrens (B) alle üblichen Säureakzeptoren eingesetzt werden. Vorzugsweise verwendbar sind Alkalimetall-und Erdalkalimetall-oxide, -hydroxide und -carbonate, wie Natriumhydroxid, Kaliumhydroxid, Magnesiumoxid, Calciumoxid, Natriumcarbonat, Kalium-carbonat und Calciumcarbonat, ferner Alkalimetall-und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat.

Als Verdünnungsmittel können bei dem erfindungsgemäßen Verfahren (B) alle üblichen inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether und weiterhin polare Lösungsmittel, wie Dimethylsulfoxid, Sulfolan, Dimethylformamid und N-Methyl-pyrrolidon.

Die Reaktionstemperaturen können bei der Durchführung des erfindungsgemäßen Verfahrens (B) innerhalb eines größeren Bereiches variiert werden. Im allgemeinen arbeitet man bei Temperaturen zwischen 50 und 200°C, vorzugsweise zwischen 100 und 180°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der

allgemeinen Formeln (IV) und (V) im allgemeinen in etwa äquimolaren Mengen ein. Es ist jedoch auch möglich, die eine oder andere Komponente in einem größeren Überschuß zu verwenden. Außerdem setzt man im allgemeinen auch eine äquimolare Menge an säurebindendem Mittel ein. Es kann jedoch auch von Vorteil sein, das Säurebindemittel in einem bis zu einmolaren Überschuß hinzuzufügen. Im einzelnen geht man im allgemeinen so vor, daß man das säurebindende Mittel zu einer Mischung der Reaktionskomponenten in einem geeigneten Verdünnungsmittel gibt. Man kann aber auch in der Weise verfahren, daß man zunächst aus dem Acylamino-Derivat der allgemeinen Formel (V) und dem Säurebindemittel ein Salz erzeugt und dieses dann mit einem Thiazol-Derivat der allgemeinen Formel (IV) umsetzt. Weiterhin ist es auch möglich, aus dem Acylamino-Derivat der allgemeinen Formel (V) mit einem Säurebindemittel zunächst separat ein Salz herzustellen, dieses dann zu isolieren und anschließend in Gegenwart eines geeigneten Verdünnungsmittels ohne weitere Zugabe eines Säurebindemittels mit einem Thiazol-Derivat der allgemeinen Formel (IV) umzusetzen. Die Aufarbeitung erfolgt jeweils nach üblichen Methoden.

Die bei dem erfindungsgemäßen Verfahren (C) als Ausgangsstoffe benötigten 2-Hydroxy bzw. (Mercapto)-thiazol-Derivate sind durch die allgemeine Formel (VI) eindeutig definiert. In dieser Formel haben $R^1$, $R^2$, und X vorzugsweise diejenigen Bedeutungen, die bereits im Zusammenhang mit der Beschreibung der erfindungsgemäßen Stoffe der allgemeinen Formel (I) vorzugsweise für diese Substituenten genannt wurden.

Die Verbindungen der allgemeinen Formel (VI) sind bekannt oder lassen sich nach bekannten Methoden herstellen. (S. hierzu: Thiazol and its derivatives Bd. II, 369 ff. in A. Weissberger, The Chemistry of heterocyclic compounds John Wiley and Sons 1979.)

Die zur Durchführung der erfindungsgemäßen Verfahren C (Variante $c_1$) und C (Variante $c_2$) weiterhin als Ausgangsstoffe benötigten 6-Halogen-nikotinsäure-Derivate sind durch die allgemeinen Formeln (VII a) und (VIIb) allgemein definiert. In den allgemeinen Formeln (VIIa) und (VIIb) haben $R^5$, $R^6$, $R^7$, $R^{5-1}$, $R^{6-1}$, D, B, $R^3$ und n vorzugsweise diejenigen Bedeutungen, die bereits bei der Beschreibung der erfindungsgemäßen Stoffe der Formel (I) vorzugsweise für diese Substituenten gennant wurden. $Hal^1$ steht vorzugsweise für Chlor oder Brom.

Die 6-Halogen-nikotinsäure-Derivate der allgemeinen Formeln (VIIa) und (VIIb) sind bekannt oder lassen sich nach bekannten Methoden herstellen.

Als Verdünnungsmittel und Säurebindemittel können bei den erfindungsgemäßen Verfahren (C-$c_1$) und (C-$c_2$) alle üblichen inerten organischen Solventien bzw. Säureakzeptoren eingesetzt werden. Vorzugsweise verwendet man die Verdünnungsmittel und Säurebindemittel, die bereits bei Verfahren (B) genannt wurden.

Die Reaktionstemperaturen können bei der Durchführung der erfindungsgemäßen Verfahren C (Variante $c_1$) und C (Variante $c_2$) innerhalb eines größeren Bereiches variiert werden. Im allgemeinen arbeitet man bei Temperaturen zwischen 80 bis 200°C, vorzugsweise bei 110 bis 180°C.

Bei der Durchführung des erfindungsgemäßen Verfahrens (C) setzt man die Reaktionskomponenten der allgemeinen Formeln (VI) und (VIIa) bzw. (VIIb) im allgemeinen in etwa äquimolaren Mengen ein. Es ist jedoch auch möglich, die eine oder andere Komponente in einem größeren Überschuß zu verwenden. Außerdem setzt man im allgemeinen auch eine äquimolare Menge an säurebindendem Mittel ein.

Die bei dem erfindungsgemäßen Verfahren (D) als Ausgangsstoffe benötigten 4-Hydroxy(Mercapto)-carbonsäure-Derivate sind durch die Formel (VIII) allgemein definiert. In der allgemeinen Formel (VIII) haben $R^3$, $R^5$, $R^6$, $R^7$, A, B, D, X und n vorzugsweise diejenigen Bedeutungen, die bereits bei der Beschreibung der erfindungsgemäßen Stoffe der allgemeinen Formel (I) vorzugsweise für diese Substituenten bzw. diesen Index genannt wurden.

Die Verbindungen der allgemeinen Formel (VIII) sind bekannt oder lassen sich nach bekannten Methoden herstellen.

Die zur Durchführung des erfindungsgemäßen Verfahrens (D) weiterhin als Ausgangsstoffe benötigten Thiazol-derivate der allgemeinen Formel (IV) wurden bereits bei der Beschreibung des Verfahrens (A) abgehandelt.

Als Verdünnungsmittel und Säurebindemittel können bei dem erfindungsgemäßen Verfahren (D) alle üblichen inerten organischen Solventien bzw. Säureakzeptoren eingesetzt werden. Vorzugsweise verwendet man die bei Verfahren (B) genannten Verdünnungsmittel und Säurebindemittel.

Die Reaktionstemperaturen können bei der Durchführung des erfindungsgemäßen Verfahrens (D) innerhalb eines größeren Bereiches variiert werden. Im allgemeinen arbeitet man bei Temperaturen zwischen 80 bis 250°C, vorzugsweise bei 110 bis 180°C.

Die erfindungsgemäßen Verfahren (C) und (D) werden im allgemeinen bei Normaldruck durchgeführt. Es ist jedoch auch möglich bei erhöhtem oder vermindertem Druck zu arbeiten.

Bei der Durchführung des erfindungsgemäßen Verfahrens (D) setzt man die Reaktionskomponenten der allgemeinen Formeln (VIII) und (IV) im allgemeinen in etwa äquimolaren Mengen ein. Es ist jedoch auch

möglich, die eine oder andere Komponente in einem größeren Überschuß zu verwenden. Außerdem setzt man im allgemeinen auch eine äquimolare Menge an säurebindendem Mittel ein.

Die zur Durchführung des erfindungsgemäßen Verfahrens (E) als Ausgangsstoffe benötigten Verbindungen der Formel ($Ia^1$) sind erfindungsgemäße Stoffe und erhältlich nach den Verfahren (A-$a_1$), (A-$a_2$), (A-$a_3$), (A-$a_4$), (B), (C-$c_1$) und (D). In der allgemeinen Formel (Ia) haben $R^1$ $R^2$, $R^3$, $R^5$, $R^6$, $R^7$, A, D, X und n vorzugsweise diejenigen Bedeutungen, die bereits bei der Beschreibung der erfindungsgemäßen Stoffe der allgemeinen Formel (I) vorzugsweise für diese Substituenten bzw. diesen Index genannt wurden.

Als Verdünnungsmittel zur Durchführung des erfindungsgemäßen Verfahrens (E) kommen inerte organische Lösungsmittel in Frage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan oder Tetrahydrofuran Die Reaktionstemperaturen können bei der Durchführung des erfindungsgemäßen Verfahrens (E) innerhalb eines größeren Bereiches variiert werden. Im allgemeinen arbeitet man bei Temperaturen zwischen +20 und +200°C, vorzugsweise zwischen 50 und 150°C.

Das erfindungsgemäße Verfahren (E) wird im allgemeinen unter Normaldruck durchgeführt. Es ist jedoch auch möglich, bei erhöhtem oder vermindertem Druck zu arbeiten.

Zur Durchführung des erfindungsgemäßen Verfahrens (E) setzt man pro Mol an Verbindung der allgemeinen Formel (Ia) 0,5 bis 5,0 Mol, vorzugsweise 0,5 bis 2,0 Mol, an Schwefelungsagentien ein.

In den bei dem erfindungsgemäßen Verfahren (F) als Ausgangsstoffe benötigten Verbindungen der allgemeinen Formel ($Ia^2$), in der D für $>$NH steht, haben $R^1$, $R^2$, $R^3$, $R^5$, $R^6$, $R^7$, A, X und n vorzugsweise diejenigen Bedeutungen, die bereits bei der Beschreibung der erfindungsgemäßen Stoffe der allgemeinen Formel (I) vorzugsweise für diese Substituenten bzw. diesen Index genannt wurden. Die Verbindungen der allgemeinen Formel ($Ia^2$), in denen D für $>$NH steht, sind erfindungsgemäße Verbindungen und erhältlich nach Verfahren (E).

Die bei dem erfindungsgemäßen Verfahren (F) weiterhin als Ausgangsstoffe benötigten Verbindungen sind durch die allgemeine Formel (IX) allgemein definiert. In der allgemeinen Formel (IX) hat $R^8$ vorzugsweise diejenige Bedeutung, die bereits bei der Beschreibung der erfindungsgemäßen Stoffe der allgemeinen Formel (I) vorzugsweise für diesen Substituenten genannt wurde. F steht vorzugsweise für Chlor, Methylsulfonyloxy, Ethylsulfonyloxy oder Phenylsulfonyloxy.

Die Verbindungen der Formel (IX) sind allgemein bekannte Verbindungen der organischen Chemie.

Als deprotonierende Basen zur Durchführung des erfindungsgemäßen Verfahrens (F) kommen alle üblichen für derartige Reaktionen verwendbaren Basen in Frage. Vorzugsweise verwendet man Alkalialkoholate wie beispielsweise Kalium-tert.-butylat, Natriummethylat, Natriumbutylat; Natrium-oder Kaliumhydroxid, Kaliumcarbonat, Natrium-oder Lithiumhydrid.

Als Verdünnungsmittel zur Durchführung des erfindungsgemäßen Verfahrens (F) kommen inerte organische Lösungsmittel in Frage. Vorzugsweise verwendet man Alkohole wie beispielsweise tert.-Butanol, Ethanol, Isopropanol, Dimethylformamid, N-Methylpyrrolidon, Dimethylsulfoxid, Sulfolan, Tetrahydrofuran oder Dioxan.

Die Reaktionstemperaturen können bei der Durchführung des erfindungsgemäßen Verfahrens (F) innerhalb eines größeren Bereiches variiert werden. Im allgemeinen arbeitet man bei Temperaturen zwischen 0 und 200°C, vorzugsweise bei 20 bis 120°C.

Das erfindungsgemäße Verfahren (F) wird im allgemeinen bei Normaldruck durchgeführt. Es ist jedoch auch möglich, bei erhöhtem oder vermindertem Druck zu arbeiten.

Die bei dem erfindungsgemäßen Verfahren G eingesetzten Thiazolderivate sind durch die allgemeine Formel ($Ia^3$) allgemein definiert. In dieser Formel haben $R^1$, $R^2$, $R^3$, X, A und n vorzugsweise diejenigen Bedeutungen, die bereits im Zusammenhang mit der Beschreibung der erfindungsgemäßen Verbindungen der allgemeinen Formel (I) vorzugsweise für diese Reste genannt wurden.

Der Rest G steht vorzugsweise für Fluor oder Chlor, für $-O-\underset{\underset{O}{\|}}{C}-OCH_3$, $-O-\underset{\underset{O}{\|}}{C}-OC_2H_5$ für $O-SO_2$-Phenyl oder

Imidazolyl.

Die Thiazolderivate der allgemeinen Formel ($Ia^3$) werden nach gängigen Verfahren der organischen Chemie aus Thiazolderivaten der allgemeinen Formel ($Ia^4$) hergestellt

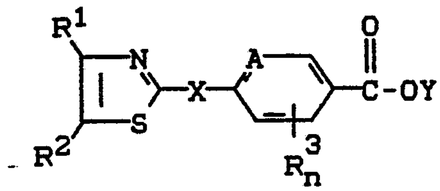

(Ia⁴)

in denen Y for Alkyl mit 1-3 C-Atomen steht und $R^1$, $R^2$, X, $R^3$, A und n die oben angegebene Bedeutung haben und die nach Verfahren D erhalten werden.

Die zur Durchführung des erfindungsgemäßen Verfahrens G weiterhin benötigten Verbindungen sind durch die Formel (XVI) allgemein definiert. In der allgemeinen Formel (XVI) stehen die Reste $R^5$, $R^6$, $R^7$ und D bevorzugt bzw. besonders bevorzugt für die Reste, die bereits im Zusammenhang mit der Beschreibung der erfindungsgemäßen Stoffe der allgemeinen Formel (I) als bevorzugt bzw. besonders bevorzugt für diesen Substituenten genannt wurden.

Die Ausgangsverbindungen der allgemeinen Formel (XVI) sind bekannte Verbindungen, wie Alkohole oder Amine oder lassen sich nach bekannten Methoden herstellen.

Als Verdünnungsmittel zur Durchführung des erfindungsgemäßen Verfahrens (G) kommen inerte organische Lösungsmittel infrage.

Vorzugsweise verwendet man Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Diozan, darüberhinaus Carbonsäureester, wie Ethylacetat, und auch stark polare Solventien, wie Dimethylsulfoxid und Sulfolan.

Für den Fall, daß G in der Formel ($Ia^3$) für Halogen steht und die Hydrolysestabilität des Säurehalogenids es zuläßt, kann die Umsetzung auch in Gegenwart von Wasser durchgeführt werden.

Verwendet man als Reaktionskomponente der allgemeinen Formel (XVI) Alkohole oder Amine in flüssiger Form, so können diese mit besonderem Vorzug in entsprechendem Überschuß gleichzeitig als Verdünnungsmittel eingesetzt werden.

Das erfindungsgemäße Verfahren (G) wird gegebenenfalls in Gegenwart eines Säurebindemittels durchgeführt. Als solche kommen alle üblichen anorganischen oder organischen Basen infrage. Vorzugsweise verwendet man tertiäre Amine, wie Triethylamin, Pyridin und N,N-Dimethylanilin, ferner Erdalkalimetalloxide, wie Magnesium-und Calciumoxid, außerdem Alkali-und Erdalkalimetall-carbonate, wie Natriumcarbonat, Kaliumcarbonat und Calciumcarbonat. Alkohole können auch in Form ihrer vorher bereiteten Metallverbindungen eingesetzt werden. Besitzt die Reaktionskomponente der allgemeinen Formel (XVI) basische Eigenschaften, so kann auch diese im entsprechenden Überschuß als Säurebindemittel eingesetzt werden.

Die Reaktionstemperaturen können bei der Durchführung des erfindungsgemäßen Verfahrens (G) innerhalb eines größeren Bereiches variiert werden. Arbeitet man ohne Lösungsmittel und Säurebindemittel, so geht man im all gemeinen so vor, daß man die Komponenten zunächst bei Temperaturen zwischen -20°C und +20°C reagieren läßt und danach auf Temperaturen zwischen 70 und 200°C erhitzt. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (G) wird im allgemeinen unter Normaldruck durchgeführt.

Zur Durchführung des erfindungsgemäßen Verfahrens (G) setzt man pro Mol Benzoesäure-Derivat der allgemeinen Formel ($Ia^3$) im allgemeinen 1,0 bis 5,0 Mol, vorzugsweise 1,0 bis 2,0 Mol, an Verbindungen der allgemeinen Formel (XVI) und gegebenenfalls 1,0 bis 3,0 vorzugsweise 1,0 bis 2,0 Mol an Säurebindemittel ein.

Bei der Durchführung des erfindungsgemäßen Verfahrens (G) ist es möglich, auf eine vorherige Herstellung der Benzoylhalogenide (in der Formel ($Ia^3$) steht G für Halogen) zu verzichten und die freien Säuren mit den Verbindungen der allgemeinen Formel (XVI) in Gegenwart eines wasserbindenden Mittels, wie beispielsweise die zur Herstellung der Benzoylhalogenide verwendbaren anorganischen Säurehalogenide, wie Phosphortrichlorid oder Phosphoroxychlorid, umzusetzen.

Die Aufarbeitung erfolgt nach üblichen Methoden. Im allgemeinen geht man bei den erfindungsgemäßen Herstellungsverfahren so vor, daß man ausgefallene Salze entfernt und das verbleibende Reaktionsgemisch durch Abziehen des Verdünnungsmittels einengt. Arbeitet man in Gegenwart von Wasser mischbaren Solventien, so kann man auch so verfahren, daß man das Reaktionsgemisch mit Wasser verdünnt, das entstehende Gemisch absaugt oder mit einem mit Wasser wenig mischbaren organischen

Lösungsmittel extrahiert, die organische Phase wäscht, einengt und den verbleibenden Rückstand gegebenenfalls üblichen Reinigungsverfahren unterwirft.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe der Formel (I) mit besonders gutem Erfolg zur selektiven Bekämpfung mono-und dikotyler Unkräuter insbesondere in monokotylen Kulturen wie beispielsweise Weizen im Nachauflaufverfahren einsetzen.

Bei der Anwendung im Nachauflaufverfahren können die erfindungsgemäßen Wirkstoffe allein oder in Kombination mit emulgierbaren Ölen, oberflächenaktiven Substanzen und anderen Additiven ausgebracht werden.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Bei der Anwendung als Herbizide können die erfindungsgemäßen Wirkstoffe als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Als Mischpartner kommen auch in Frage:

Harnstoffe (z.B. Methabenzthiazuron, Chlortuluron, Isoproturon), Sulfonylharnstoffe (z.B. Chlorsufuron), Triazine (z.B. Atrazin, Terbutryne, Cyanazin), Triazinone (z.B. Ethiozin, Metribuzin), Triazindione (z.B. Amethydione), Diphenylether (z.B. Bifenox), Benzonitrile (z.B. Ioxymil, Bromoxymil), Phenoxyalkancarbonsäuren (z.B. 2,4 D, 2,4 DP, MCPA, MCPP etc.), Aryloxy-oder Heteroaryloxyphenoxypropionsäuren (z.B. Illoxan, Whip, (R)-2-[4-(3,5-Dichlor-pyridyl-2-oxy)-phenoxy]-propionsäure-(trimethylsilyl)-methylester, Imidazolinone (z.B. Imazamethabenz), Bentazone und Pyridate.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

<u>Herstellungsbeispiele:</u>

Beispiel 1:

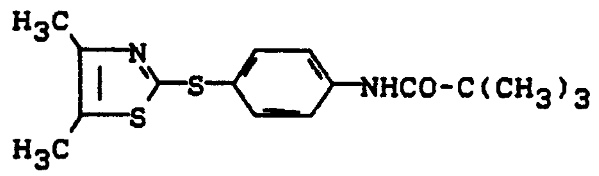

(I-1)

**(Verfahren A-a$_1$)**

23.6 g (0.1 mol) 2-(4-Aminophenylthio)-4,5-dimethylthiazol werden in 200 ml Toluol gelöst. Hierzu tropft man 10 g (0.1 mol) Triethylamin und 12 g (0.1 mol) Pivalsäurechlorid. Das Gemisch wird zwei Stunden bei Raumtemperatur nachgerührt. Nach Zugabe von 200 ml Wasser wird der entstandene Feststoff abgesaugt, mit Wasser gewaschen und getrocknet. Man erhält 21.7 g des gewünschten Amids. Fp. 137°C. (68 % der Theorie).

Beispiel 2

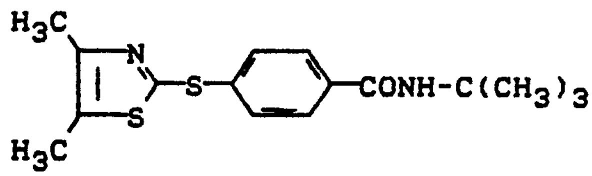

(I-2)

**(Verfahren D)**

Zu 6.3 g (0.03 mol) 4-Mercaptobenzoesäure-t-butylamid in 80 ml abs. DMF werden bei Raumtemperatur 0.9 g (0.03 mol) 80%iges Natriumhydrid gegeben. Nach beendeter Wasserstoffentwicklung werden 4.4 g (0.03 mol) 2-Chlor-4,5-dimethylthiazol zugetropft. Das Reaktionsgemisch wird 6 Stunden zum Rückfluß erhitzt, abgekühlt und in 500 ml Eiswasser eingerührt. Das gebildete Produkt wird abgesaugt und getrocknet. Man erhält 8.3 g (86.4% d. Theorie) des gewünschten Thiazols.
Fp: 109°C.

Beispiel 3:

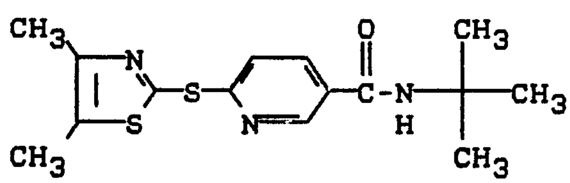

(I-3)

**(Verfahren C-c$_1$)**

14.51 g (0.1 Mol) 4.5-Dimethyl-2-mercapto-thiazol werden in 80 ml N-Methyl-pyrrolidon unter Stickstoffatmosphäre mit 6.72 g (0.12 Mol) gepulverter KOH versetzt, 15 Minuten verrrührt und nach Zugabe von 21 g (0.1 Mol) 6-Chlornicotinsäure-tert.-butylamid 5 h auf 150°C erwärmt. Es wird in 400 ml eisgekühlte 1N NaOH eingegossen und abgesaugt. Nach Umkristallisieren aus Toluol/n-Hexan werden 12.71 g (39.6% der Theorie) 6-(4.5-Dimethyl-2-mercapto-thiazolyl)-nicotinsäure-tert.-butylamid erhalten.
Fp.: 111°C

Beispiel 4:

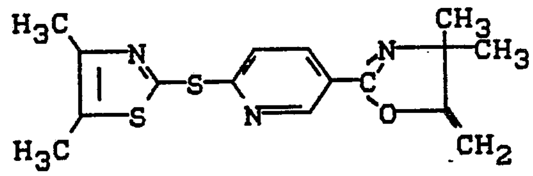

(I-4)

(Verfahren $C-c_2$)

4,35 g (30 mmol) 4,5-Dimethyl-2-mercapto-thiazol werden in 30 ml N-Methylpyrrolidon unter Stickstoffatomsphäre mit 2,02 g (36 mmol) gepulverter KOH versetzt, 15 Min. verrührt und nach Zugabe von 6.67 g (30 mmol) 6-Chlornicotinsäure-dimethyl-propargylamid 5 h auf 150°C erwärmt. Es wird in 200 ml Eiswasser eingerührt und abgesaugt. Nach zweimaliger Kristallisation aus Essigester/n-Hexan erhält man 2.94 g (30.3 % der Theorie).
Fp.: 92°C

Beispiel 5:

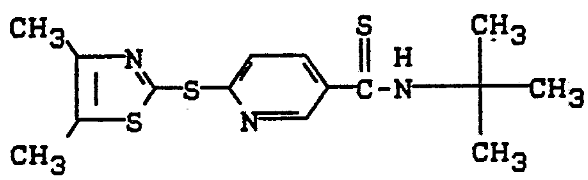

(I-5)

(Verfahren E )

9 g (28 mmol) 6-(4,5-Dimethyl-2-mercapto-thiazolyl)-nicotinsäure-tert-butylamid und 6.16 g (15.4 mmol) Lawesson-Reagenz werden in 28 ml Toluol unter DC-Kontrolle rückflussiert. Der Rückstand wird an Kieselgel mit Toluol/Aceton 9:1 chromatographiert und das Produkt aus Essigester/n-Hexan kristallisiert. Man erhält 4.48 g (47.5 % der Theorie) 6-(4,5-Dimethyl-2-mercapto-thiazolyl)-nicotinsäure-tert. butylthioamid vom Schmelzpunkt 120°C.

Analog werden nach einem der beschriebenen Verfahren die folgenden Verbindungen der allgemeinen Formel (I)

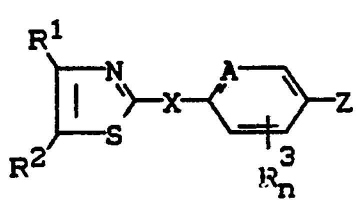

(I)

hergestellt.

Tabelle 1:

| Bsp. Nr. | $R^1$ | $R^2$ | $R^3$ | A | X | Z | Phys. Daten [Fp/°C, $n_D^{20}$] |
|---|---|---|---|---|---|---|---|
| 6 | $-CH_3$ | H | H | =CH- | S | $-CONHC_4H_9$ (t) | 177 |
| 7 | $-CH_3$ | $-CH_3$ | H | =CH- | O | $-CONHC_4H_9$ (t) | 115 |
| 8 | $-CH_3$ | $-CH_3$ | H | =CH- | S | $-NHCSC_4H_9$ (t) | 125 |
| 9 | $-C_4H_9$ (t) | H | H | =CH- | S | $-CONHC_4H_9$ (t) | 130 |
| 10 | $-C_2H_5$ | $-CH_3$ | H | =CH- | S | $-CONHC_4H_9$ (t) | 116 |
| 11 | $-C_2H_5$ | H | H | =CH- | S | $-CONHC_4H_9$ (t) | 125 |
| 12 | $-C_3H_7$(i) | H | H | =CH- | S | $-CONHC_4H_9$ (t) | 111 |
| 13 | $-CH_2-C_4H_9$(t) | H | H | =CH- | S | $-CONHC_4H_9$ (t) | 117 |
| 14 | $-CH_3$ | $-C_3H_7$(i) | H | =CH- | S | $-CONHC_4H_9$ (t) | 100 |
| 15 | $-C_3H_7$(i) | $-CH_3$ | H | =CH- | S | $-CONHC_4H_9$ (t) | 118 |
| 16 | $-CH_3$ | $-CH_3$ | H | =CH- | S | 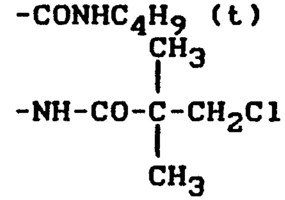 | 124 |
| 17 | $-CH_3$ | $-CH_3$ | H | =CH- | S | 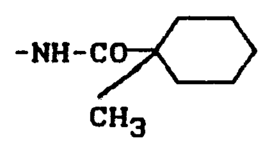 | 118 |

<u>Tabelle 1</u>: (Fortsetzung)

| Bsp. Nr. | $R^1$ | $R^2$ | $R^3$ | A | X | Z | Phys. Daten [Fp/°C, $n_D^{20}$] |
|---|---|---|---|---|---|---|---|
| 18 | $-CH_3$ | $-CH_3$ | H | =CH- | S | 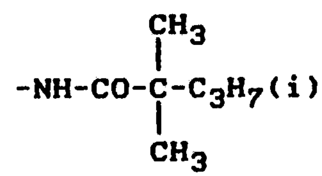 | 1.6915 |
| 19 | $-CH_3$ | $-CH_3$ | H | =CH- | S | 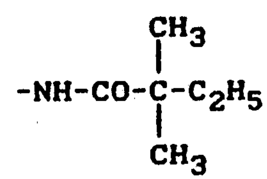 | 122 |
| 20 | $-CH_3$ | $-CH_3$ | H | =CH- | S | 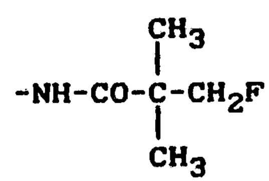 | 114 |
| 21 | $-CH_3$ | $-CH_3$ | Cl 1) | =CH- | S | $-COOC_2H_5$ | 1.6950 |

<u>Tabelle 1</u>: (Fortsetzung)

| Bsp. Nr. | $R^1$ | $R^2$ | $R^3$ | A | X | Z | Phys. Daten [Fp/°C, $n_D^{20}$] |
|---|---|---|---|---|---|---|---|
| 22 | $-CH_3$ | $-CH_3$ | H | =CH- | S | $CH_3$<br>-NH-CO-C-$CH_2F$<br>$CH_2F$ | 96 |
| 23 | $-CH_3$ | $-CH_3$ | H | =CH- | S | $-COOC_2H_5$ | 88 |
| 24 | $-CH_3$ | $-CH_3$ | H | =CH- | S | $-NH-CO-CH_2C_4H_9(t)$ | 122 |
| 25 | $-CH_3$ | $-CH_3$ | H | =N- | S | $CH_3$<br>-CONH-C-$CH_2C_4H_9(t)$<br>$CH_3$ | 112 |

Tabelle 1: (Fortsetzung)

| Bsp. Nr. | $R^1$ | $R^2$ | $R^3$ | A | X | Z | Phys. Daten [Fp/°C, $n_D^{20}$] |
|---|---|---|---|---|---|---|---|
| 26 | $-CH_3$ | $-CH_3$ | H | =N- | S | 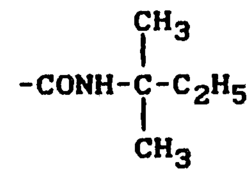 | 92 |
| 27 | $-CH_3$ | $-CH_3$ | H | =N- | S | $-COOC_4H_9(t)$ | 55 |
| 28 | $-CH_3$ | $-CH_3$ | H | =N- | S | 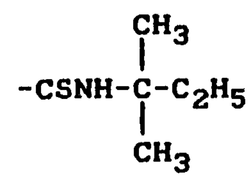 | 153 |
| 29 | $-CH_3$ | $-CH_3$ | H | =CH- | S | 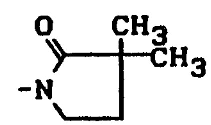 | 57 |

Tabelle 1: (Fortsetzung)

| Bsp. Nr. | $R^1$ | $R^2$ | $R^3$ | A | X | Z | Phys. Daten [Fp/°C, $n_D^{20}$] |
|---|---|---|---|---|---|---|---|
| 30 | $-CH_3$ | $-CH_3$ | H | =N- | S | 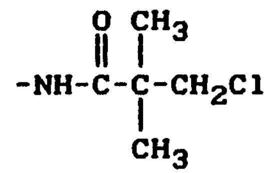 | 120 |
| 31 | $-CH_3$ | $-CH_3$ | H | =N- | S | 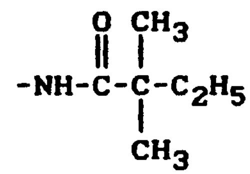 | 109 |
| 32 | $-CH_3$ | $-CH_3$ | H | =N- | S | 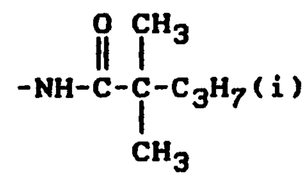 | 101 |
| 33 | $-CH_3$ | $-CH_3$ | H | =N- | S | 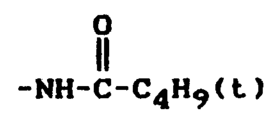 | 98 |

<u>Tabelle 1</u>: (Fortsetzung)

| Bsp. Nr. | $R^1$ | $R^2$ | $R^3$ | A | X | Z | Phys. Daten [Fp/°C, $n_D^{20}$] |
|---|---|---|---|---|---|---|---|
| 34 | $-CH_3$ | $-CH_3$ | H | =N- | S | 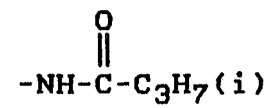 | 106 |
| 35 | $-CH_3$ | $-CH_3$ | H | =N- | S | 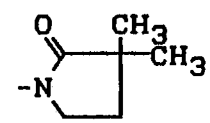 | 43 |
| 36 | $-CH_3$ | $-CH_3$ | H | =CH- | S | 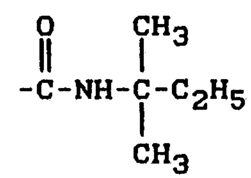 | 73 |
| 37 | $-CH_3$ | $-CH_3$ | H | =CH- | S | 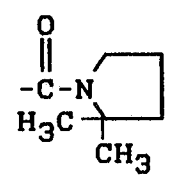 | 82 |
| 38 | $-CH_3$ | $-CH_3$ | H | =CH- | S | $-CO-O-C(CH_3)_3$ | Öl |

**Tabelle 1:** (Fortsetzung)

| Bsp. Nr. | $R^1$ | $R^2$ | $R^3$ | A | X | Z | Phys. Daten [Fp/°C, $n_D^{20}$] |
|---|---|---|---|---|---|---|---|
| 39 | $-CH_3$ | $-CH_3$ | H | =N- | S | $-C=N-C(CH_3)_3$<br>\|<br>$SCH_3$ | Öl |
| 40 | $-CH_3$ | $-CH_3$ | H | =N- | S | $-C=N-C(CH_3)_3$<br>\|<br>$S-CH_2-C=CH_2$ | Öl |
| 41 | $-CH_3$ | $-CH_3$ | H | =N- | S | $-C=N-C(CH_3)_3$<br>\|<br>$S-C_2H_5$ | Öl |
| 42 | H | $-CH_3$ | H | =CH- | S | $-CO-NH-C(CH_3)_3$ | 98 |

1) Stellung: ortho-ständig zu Z

Herstellung von Ausgangsstoffen:

Beispiel a

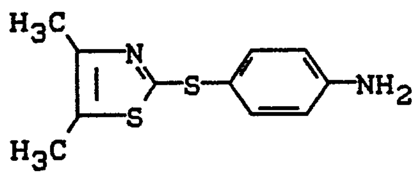

21.1 g (0.08 mol) 2-(4-Nitrophenylthio)-4,5-dimethylthiazol werden in 200 ml Dioxan mit Wasserstoff in Gegenwart von 5 g Ra-Nickel unter einem Druck von 50 bar bei einer Temperatur zwischen 20 und 70°C erschöpfend hydriert. Nach Abfiltrieren des Katalysators wird das Lösungsmittel im Vakuum abdestilliert und der verbleibende Rückstand an Kieselgel mit einem Gemisch Essigester/Cyclohexan (1:1) chromatographiert. Man erhält so 13.6 g (72% der Theorie) des gewünschten Aminoderivates;
Fp.: 68°C.

Herstellung des Vorproduktes

50 g (0.34 mol) 2-Mercapto-4,5-dimethylthiazol werden unter Zugabe von 19.6 g (0.35 mol) gepulverten Kaliumhydroxids in 500 ml Sulfolan gelöst und nach 30 minütigem Rühren mit 55.1 g (0.35 ml) 4-Chlornitrobenzol versetzt. Das Reaktionsgemisch wird 4 Stunden auf 130°C erhitzt, abgekühlt und in 2 l Eiswasser eingerührt. Nach Absaugen und Trocknen des Niederschlages erhält man 85 g (94% der Theorie) der gewünschten Nitroverbindung.
Fp.: 77°C.

Verwendungsbeispiel

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die erfindungsgemäßen Verbindungen I-2, I-7, I-16, I-17 und I-18 eine sehr gute herbizide Wirkung.

**Ansprüche**

1. Thiazolylderivate der allgemeinen Formel (I)

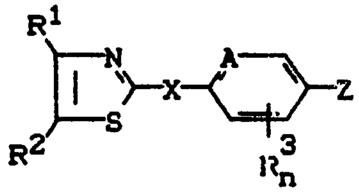

(I)

in welcher
$R^1$ für Wasserstoff oder Alkyl mit 1-6 Kohlenstoffatomen steht,
$R^2$ für Wasserstoff oder Alkyl mit 1-6 Kohlenstoffatomen steht,
mit der Maßgabe, daß $R^1$ und $R^2$ nicht gleichzeitig für Wasserstoff stehen,
A für eine (-CH=)Gruppe oder ein Stickstoffatom steht,
$R^3$ für Wasserstoff, Halogen, Cyano, Alkyl bzw. Alkoxy mit jeweils 1-6 Kohlenstoffatomen steht, wobei die Reste $R^3$ gleich oder verschieden sein können,
n für eine Zahl von 1-3 steht
X für Sauerstoff oder Schwefel steht
Z für den Rest

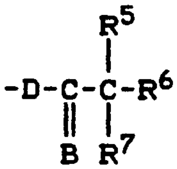

oder

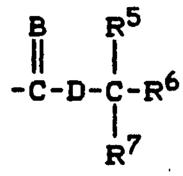

(Ia₁) (Ia₂)

oder

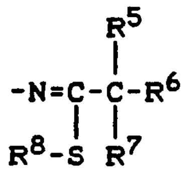

oder

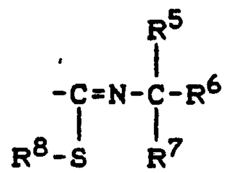

(Ib₁) (Ib₂)

oder

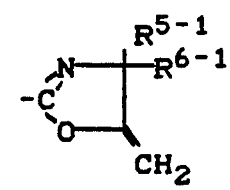

(Ic₁)

steht,
wobei
B für Sauerstoff oder Schwefel steht,
D für >N-$R^4$ oder Sauerstoff steht, wobei
$R^4$ für Wasserstoff, Alkyl mit 1-6 Kohlenstoffatomen oder Alkoxyalkyl mit jeweils 1 bis 6 Kohlenstoffatomen je Alkylteil, für Alkenyl oder Alkinyl mit jeweils 3 bis 6 Kohlenstoffatomen steht,
$R^5$, $R^6$ und $R^7$ jeweils unabhängig voneinander für Wasserstoff, Halogen, Cyano, Alkyl mit 1 bis 6 Kohlenstoffatomen, Halogenalkyl mit 1 bis 6 Kohlenstoffatomen und bis zu 13 Halogenatomen, Alkoxyalkyl

mit jeweils 1 bis 6 Kohlenstoffatomen je Alkylteil, Alkenyl oder Alkinyl mit jeweils 2 bis 6 Kohlenstoffatomen stehen,
$R^4$ und $R^5$ gemeinsam mit dem Kohlenstoff und dem Stickstoff, an die sie gebunden sind, einen 3 bis 8 gliedrigen heterocyclischen Ring bilden, der gegebenenfalls einfach oder mehrfach, gleich oder verschieden durch Halogen, Methyl oder Ethyl substituiert ist und welcher eine oder mehrere Doppelbindungen enthalten kann,
$R^6$ und $R^7$ gemeinsam mit dem Kohlenstoffatom, an das sie gebunden sind, einen 3 bis 8 gliedrigen Ring bilden, der außer Kohlenstoff auch Sauerstoff-und/oder Schwefelatome als Ringglieder enthalten kann und der gegebenenfalls einfach oder mehrfach, gleich oder verschieden durch Halogen, Methyl und Ethyl substituiert ist und welcher eine oder mehrere Doppelbindungen enthalten kann und
$R^8$ für gegebenenfalls einfach oder mehrfach, gleich oder verschieden durch Halogen substituiertes Alkyl mit 1 bis 6 Kohlenstoffatomen, für Alkoxyalkyl mit 1 bis 6 Kohlenstoffatomen je Alkylteil, für Alkenyl oder Alkinyl mit jeweils 3 bis 6 Kohlenstoffatomen steht, und
$R^{5-1}$ und $R^{6-1}$ unabhängig voneinander jeweils für Alkyl mit 1-6 Kohlenstoffatomen stehen.

2. Verbindungen der allgemeinen Formel (I) gemäß Anspruch 1, in welchen
$R^1$ für Wasserstoff oder Alkyl mit 1-4 Kohlenstoffatomen steht,
$R^2$ für Wasserstoff oder Alkyl mit 1-4 Kohlenstoffatomen steht,
mit der Maßgabe, daß $R^1$ und $R^2$ nicht gleichzeitig für Wasserstoff stehen,
A für eine (-CH=)Gruppe oder ein Stickstoffatom steht,
$R^3$ für Wasserstoff, Fluor, Chlor, Brom, Iod, Cyano, für Alkyl oder Alkoxy mit jeweils 1 bis 4 Kohlenstoffatomen steht, wobei die Reste $R^3$ gleich oder verschieden sein können
n für eine Zahl von 1 bis 3 steht,
X für Sauerstoff oder Schwefel steht
Z für den Rest

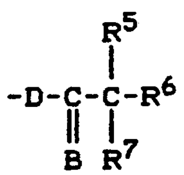

oder

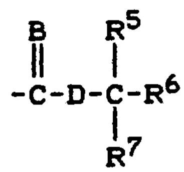

$(Ia_1)$ $(Ia_2)$

oder

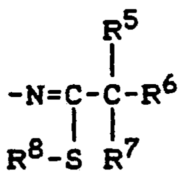

oder

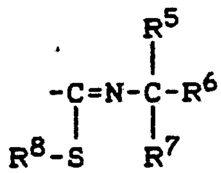

$(Ib_1)$ $(Ib_2)$

oder

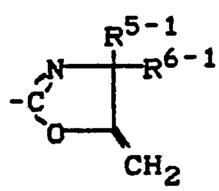

$(Ic_1)$

steht,
wobei
B für Sauerstoff oder Schwefel steht,
D für >N-$R^4$ oder Sauerstoff steht, wobei
$R^4$ für Wasserstoff, Alkyl mit 1-4 Kohlenstoffatomen oder Alkoxyalkyl mit jeweils 1 bis 4 Kohlenstoffatomen je Alkylteil, für Alkenyl oder Alkinyl mit jeweils 3 bis 4 Kohlenstoffatomen steht,

$R^5$, $R^6$ und $R^7$ jeweils unabhängig voneinander für Wasserstoff, Fluor, Chlor, Brom, Iod, Cyano, Alkyl mit 1 bis 5 Kohlenstoffatomen, Halogenalkyl mit 1 bis 4 Kohlenstoffatomen und 1 bis 9 Halogenatomen, Alkoxyalkyl mit jeweils 1 bis 4 Kohlenstoffatomen je Alkylteil, Alkenyl oder Alkinyl mit jeweils 2 bis 4 Kohlenstoffatomen stehen,

$R^4$ und $R^5$ gemeinsam mit dem Kohlenstoff und dem Stickstoff, an die sie gebunden sind, einen 3 bis 7 gliedrigen heterocyclischen Ring bilden, der gegebenenfalls einfach bis zehnfach, gleich oder verschieden durch Fluor, Chlor, Brom, Methyl oder Ethyl substituiert ist und welcher eine bis zwei Doppelbindungen enthalten kann.

$R^6$ und $R^7$ gemeinsam mit dem Kohlenstoffatom, an das sie gebunden sind, einen 3 bis 7 gliedrigen Ring bilden, der außer Kohlenstoff auch Sauerstoff-und/oder Schwefelatome als Ringglieder enthalten kann und der gegebenenfalls einfach bis zwölffach, gleich oder verschieden durch Fluor, Chlor, Brom, Methyl oder Ethyl substituiert ist und welcher eine bis drei Doppelbindungen enthalten kann und

$R^8$ für gegebenenfalls einfach bis fünffach, gleich oder verschieden durch Fluor, Chlor oder Brom substituiertes Alkyl mit 1 bis 5 Kohlenstoffatomen, für Alkoxyalkyl mit 1 bis 5 Kohlenstoffatomen je Alkylteil, für Alkenyl oder Alkinyl mit jeweils 3 bis 4 Kohlenstoffatomen steht, und

$R^{5-1}$ und $R^{6-1}$ unabhängig voneinander jeweils für Alkyl mit 1-4 Kohlenstoffatomen stehen.

3. Verbindungen der allgemeinen Formel (I) gemäß Anspruch 1, in welchen

$R^1$ für Wasserstoff, Methyl oder Ethyl steht und

$R^2$ für Wasserstoff oder Alkyl mit 1-3 Kohlenstoffatomen steht,

mit der Maßgabe, daß $R^1$ und $R^2$ nicht gleichzeitig für Wasserstoff stehen,

A für eine (-CH=)Gruppe oder ein Stickstoffatom steht

$R^3$ für Wasserstoff, Fluor, Chlor, Cyano, Methyl, Ethyl, Methoxy oder Ethoxy steht, wobei die Reste $R^3$ gleich oder verschieden sein können

n für die Zahl 1-3 steht

X für Sauerstoff oder Schwefel steht

Z für den Rest

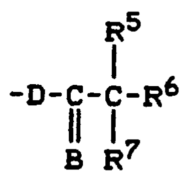

oder

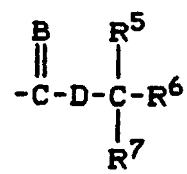

(Ia1) (Ia2)

oder

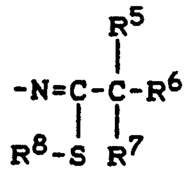

oder

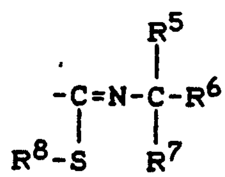

(Ib1) (Ib2)

oder

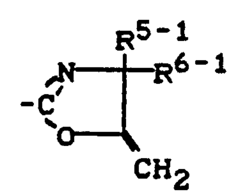

(Ic1)

steht,

wobei

B für Sauerstoff oder Schwefel steht,

D für >N-$R^4$ oder Sauerstoff steht, wobei

$R^4$ für Wasserstoff, Methyl, Ethyl, Methoxymethyl, Methoxyethyl, Ethoxymethyl, Ethoxyethyl, Allyl oder Propargyl steht,
$R^5$ und $R^6$ unabhängig voneinander für Wasserstoff, Fluor, Chlor, Brom, Methyl, Ethyl, n-Propyl, iso-Propyl, Halogen-$C_1$-$C_4$-alkyl mit 1 bis 5 gleichen oder verschiedenen Fluor-und Chloratomen steht,
$R^7$ für Wasserstoff, Fluor, Chlor, Brom, Cyano, Methyl, Ethyl, n-Propyl, iso-Propyl, n-Butyl, iso-Butyl, sec.-Butyl, tert.-Butyl, n-Pentyl, iso-Pentyl, neo-Pentyl, tert.-Pentyl, Alkoxyalkyl mit jeweils 1 bis 3 Kohlenstoffatomen je Alkylteil, Vinyl, Allyl, Ethinyl, Propargyl, Halogen-$C_1$-$C_4$-alkyl mit 1 bis 5 gleichen oder verschiedenen Fluor-und Chloratomen steht oder
$R^4$ und $R^5$ gemeinsam mit dem Kohlenstoff und dem Stickstoff, an die sie gebunden sind, einen 3 bis 6 gliedrigen heterocyclischen Ring bilden, der gegebenenfalls einfach bis achtfach, gleich oder verschieden durch Fluor, Chlor, Methyl und Ethyl substituiert ist und welcher eine oder zwei Doppelbindungen enthalten kann, oder
$R^6$ und $R^7$ gemeinsam mit dem Kohlenstoff, an den sie gebunden sind, einen 3 bis 6 gliedrigen Ring bilden, der außer Kohlenstoff auch Sauerstoff und/oder Schwefel als Ringglieder enthalten kann und der gegebenenfalls einfach bis zehnfach, gleich oder verschieden durch Fluor, Chlor, Methyl oder Ethyl substituiert ist und der eine bis zwei Doppelbindungen enthalten kann, und
$R^8$ für gegebenenfalls einfach bis dreifach, gleich oder verschieden durch Fluor, Chlor und Brom substituiertes $C_1$-$C_2$-Alkyl, Methoxymethyl, Methoxyethyl, Ethoxymethyl, Ethoxyethyl, Allyl oder Propargyl steht und
$R^{5-1}$ und $R^{6-1}$ unabhängig voneinander für Methyl, Ethyl oder iso-Propyl steht.

4. Verfahren zur Herstellung von Thiazolylderivaten der allgemeinen Formel (I)

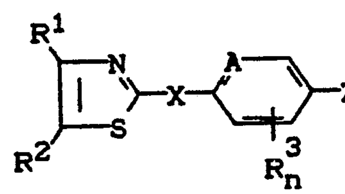

(I)

in welcher
$R^1$ für Wasserstoff oder Alkyl mit 1-6 Kohlenstoffatomen steht,
$R^2$ für Wasserstoff oder Alkyl mit 1-6 Kohlenstoffatomen steht,
mit der Maßgabe, daß $R^1$ und $R^2$ nicht gleichzeitig für Wasserstoff stehen,
A für eine (-CH=)Gruppe oder ein Stickstoffatom steht,
$R^3$ für Wasserstoff, Halogen, Cyano, Alkyl bzw. Alkoxy mit jeweils 1-6 Kohlenstoffatomen steht, wobei die Reste $R^3$ gleich oder verschieden sein können
n für eine Zahl von 1-3 steht
X für Sauerstoff oder Schwefel steht
Z für den Rest

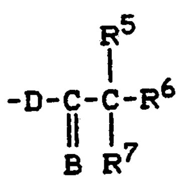

(Ia$_1$)

oder

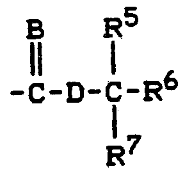

(Ia$_2$)

oder

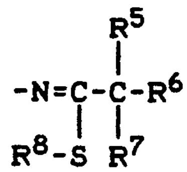 oder 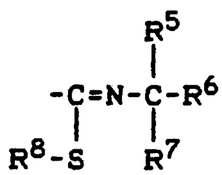

$(Ib_1)$ $(Ib_2)$

oder 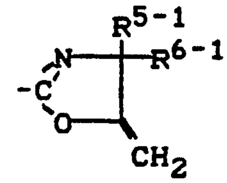

$(Ic_1)$

steht,
wobei
B für Sauerstoff oder Schwefel steht,
D für >N-$R^4$ oder Sauerstoff steht, wobei
$R^4$ für Wasserstoff, Alkyl mit 1-6 Kohlenstoffatomen oder Alkoxyalkyl mit jeweils 1 bis 6 Kohlenstoffatomen je Alkylteil, für Alkenyl oder Alkinyl mit jeweils 3 bis 6 Kohlenstoffatomen steht,
$R^5$, $R^6$ und $R^7$ jeweils unabhängig voneinander für Wasserstoff, Halogen, Cyano, Alkyl mit 1 bis 6 Kohlenstoffatomen, Halogenalkyl mit 1 bis 6 Kohlenstoffatomen und bis zu 13 Halogenatomen, Alkoxyalkyl mit jeweils 1 bis 6 Kohlenstoffatomen je Alkylteil, Alkenyl oder Alkinyl mit jeweils 2 bis 6 Kohlenstoffatomen stehen,
$R^4$ und $R^5$ gemeinsam mit dem Kohlenstoff und dem Stickstoff, an die sie gebunden sind, einen 3 bis 8 gliedrigen heterocyclischen Ring bilden, der gegebenenfalls einfach oder mehrfach, gleich oder verschieden durch Halogen, Methyl oder Ethyl substituiert ist und welcher eine oder mehrere Doppelbindungen enthalten kann.
$R^6$ und $R^7$ gemeinsam mit dem Kohlenstoffatom, an das sie gebunden sind, einen 3 bis 8 gliedrigen Ring bilden, der außer Kohlenstoff auch Sauerstoff-und/oder Schwefelatome als Ringglieder enthalten kann und der gegebenenfalls einfach oder mehrfach, gleich oder verschieden durch Halogen, Methyl und Ethyl substituiert ist und welcher eine oder mehrere Doppelbindungen enthalten kann und
$R^8$ für gegebenenfalls einfach oder mehrfach, gleich oder verschieden durch Halogen substituiertes Alkyl mit 1 bis 6 Kohlenstoffatomen, für Alkoxyalkyl mit 1 bis 6 Kohlenstoffatomen je Alkylteil, für Alkenyl oder Alkinyl mit jeweils 3 bis 6 Kohlenstoffatomen steht, und
$R^{5-1}$ und $R^{6-1}$ unabhängig voneinander jeweils für Alkyl mit 1-6 Kohlenstoffatomen stehen,
dadurch gekennzeichnet, daß

A) Verbindungen der allgemeinen Formel (II)

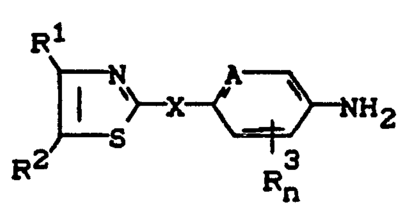 (II)

in welcher
$R^1$, $R^2$, $R^3$, n, X und A die oben angegebene Bedeutung haben, entweder

$a_1$) mit Säurehalogeniden der Formel (IIIa),

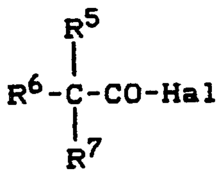

(IIIa)

in welcher
$R^5$, $R^6$, $R^7$ die oben angegebene Bedeutung haben und
Hal für Halogen steht,
gegebenenfalls in Gegenwart eines Verdünnungsmittels und gegebenenfalls in Gegenwart eines Säurebindemittels umsetzt,
oder

$a_2$) mit symmetrischen Carbonsäureanhydriden der Formel (IIIb),

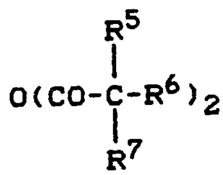

(IIIb)

in welcher
$R^5$, $R^6$ und $R^7$ die oben angegebene Bedeutung haben,
gegebenenfalls in Gegenwart eines Verdünnungsmittels umsetzt,
oder

$a_3$) mit asymmetrischen Säureanhydriden der Formel (IIIc),

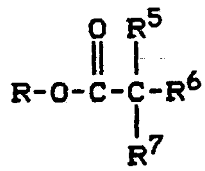

(IIIc)

in welcher
$R^5$, $R^6$ und $R^7$ die oben angegebene Bedeutung haben und
R für Alkoxycarbonyl, Alkylsulfonyl, Phenoxycarbonyl oder Phenylsulfonyl steht,
gegebenenfalls in Gegenwart eines Verdünnungsmittels umsetzt,
oder

$a_4$) mit Verbindungen der Formel (IIId),

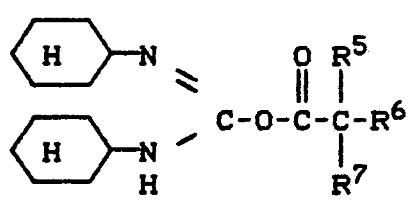

(IIId)

in welcher
$R^5$, $R^6$ und $R^7$ die oben angegebene Bedeutung haben,
gegebenenfalls in Gegenwart eines Verdünnungsmittels umsetzt,
oder daß man

B) Thiazolderivate der allgemeinen Formel (IV),

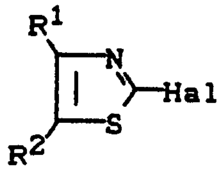

(IV)

in welcher
$R^1$ und $R^2$ die oben angegebene Bedeutung haben und
Hal für Halogen steht,
mit Verbindungen der allgemeinen Formel (V)

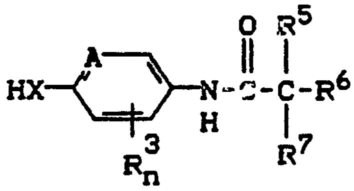

(V)

in welcher
$R^3$, $R^5$, $R^6$, $R^7$, X, A und n die oben angegebene Bedeutung haben,
gegebenenfalls in Gegenwart eines Verdünnungsmittels und gegebenenfalls in Gegenwart eines Säurebindemittels umsetzt,
oder daß man

C) Thiazol-Derivate der allgemeinen Formel (VI),

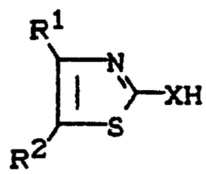

(VI)

in welcher
$R^1$, $R^2$ und X die oben angegebene Bedeutung haben,
entweder

$c_1$) mit 2-Halogen-pyridinderivaten der allgemeinen Formel (VIIa),

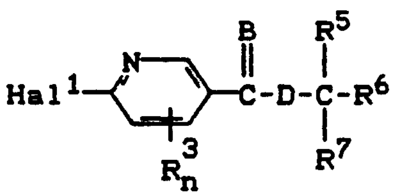

(VIIa)

in welcher
$R^3$, $R^5$, $R^6$, $R^7$, B, D und n die oben angegebene Bedeutung haben
$Hal^1$ für Halogen steht,
gegebenenfalls in Gegenwart eines Verdünnungsmittels und gegebenenfalls in Gegenwart eines Säurebindemittels umsetzt,
oder

$c_2$) mit 6-Halogennikotinsäurederivaten der allgemeinen Formel (VIIb),

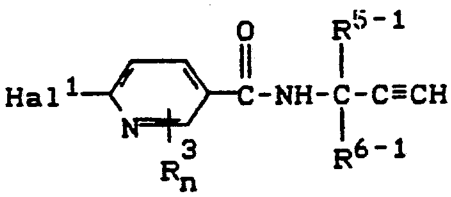

(VIIb)

in welcher
$R^3$, $R^{5-1}$, $R^{6-1}$, n und $Hal^1$ die oben angegebene Bedeutung haben
gegebenenfalls in Gegenwart eines Verdünnungsmittels und in Gegenwart einer Base umsetzt zu den Verbindungen der allgemeinen Formel ($Ic_1$)

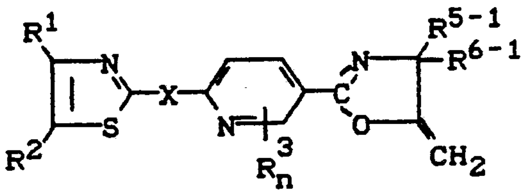

($Ic_1$)

in welcher
$R^1$, $R^2$, $R^3$, X, n, $R^{5-1}$ und $R^{6-1}$ die oben angebene Bedeutung haben,
oder, daß man

D) Verbindungen der allgemeinen Formel (VIII),

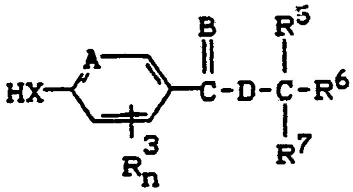

(VIII)

in welcher
$R^3$, $R^5$, $R^6$, $R^7$, A, B, D, X und n die oben angegebene Bedeutung haben,
mit Thiazol-Derivaten der allgemeinen Formel (IV),

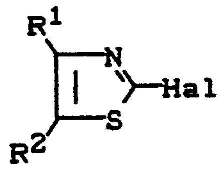

(IV)

in welcher
$R^1$ und $R^2$ die oben angegene Bedeutung haben und
Hal für Halogen steht,
gegebenenfalls in Gegenwart eines Verdünnungsmittels und gegebenenfalls in Gegenwart eines Säurebindemittels umsetzt,
oder, daß man

E) substituierte Thiazolylderivate der allgemeinen Formel ($Ia^1$)

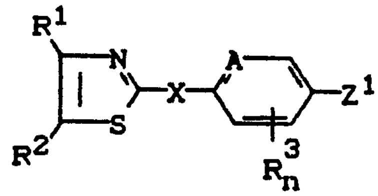

(Ia¹)

in welcher
$Z^1$ für den Rest

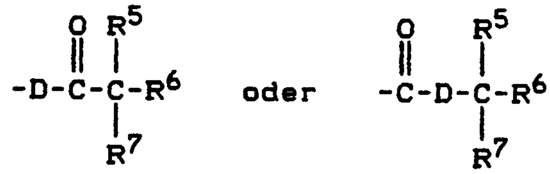

steht und
$R^1$, $R^2$, $R^3$, $R^5$, $R^6$, $R^7$, A, D, X und n die oben angegebene Bedeutung haben,
mit Schwefelungsreagenzien, wie beispielsweise Phosphor-V-sulfid oder 2,4-Bis-(4-methoxyphenyl)-2,4-dithiono-1,2,3,4-dithia-phosphetan (Lawesson-Reagenz) umsetzt zu Verbindungen der allgemeinen Formel ($Ia^2$)

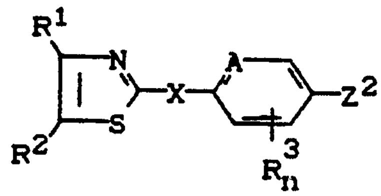

(Ia²)

in welcher
$Z^2$ für den Rest

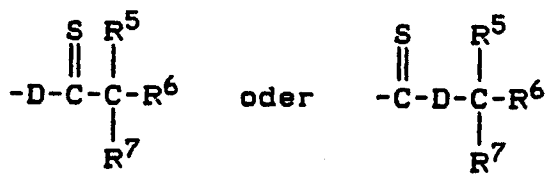

steht und
$R^1$, $R^2$, $R^3$, $R^5$, $R^6$, $R^7$, A, D, X und n die oben angegebene Bedeutung haben,
oder, daß man

F) die nach dem Verfahren (E) hergestellten Verbindungen der allgemeinen Formel ($Ia^2$), in denen D für >NH steht, mit deprotonierenden Basen und Verbindungen der allgemeinen Formel (IX),

E-$R^8$ (IX)

in welcher
$R^8$ die oben angegebene Bedeutung hat und
E für Halogen, Alkylsulfonyloxy oder Phenylsulfonyloxy steht,
gegebenenfalls in Gegenwart eines Verdünnungsmittels zu Verbindungen der allgemeinen Formeln ($Ib_1$) oder ($Ib_2$) umsetzt

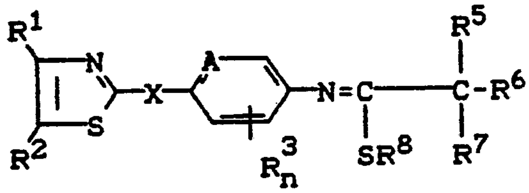

(Ib$_1$)

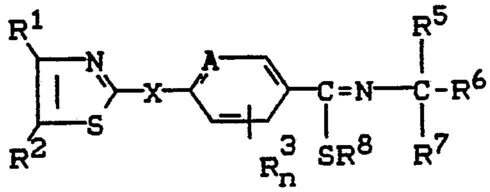

(Ib$_2$)

in denen
A, $R^1$, $R^2$, $R^3$, $R^5$, $R^6$, $R^7$, $R^8$, X und n die oben angegebene Bedeutung haben
oder daß man

G) Verbindungen der allgemeinen Formel (Ia$^3$)

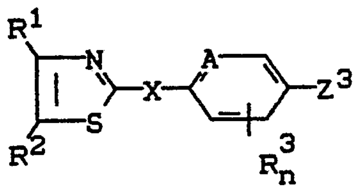

(Ia$^3$)

in welcher
$R^1$, $R^2$, $R^3$, X, A und n die oben angegebene Bedeutung haben und $Z^3$ für den Rest

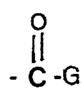

und G für Halogen, für den Rest

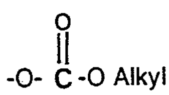

oder -O-$SO_2$-Aryl oder Imidazolyl steht, mit Verbindungen der allgemeinen Formel (XVI)

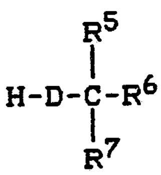

(XVI)

in welcher
D, $R^5$, $R^6$ und $R^7$ die oben angegebene Bedeutung haben gegebenenfalls in Gegenwart eines Verdünnungsmittels und gegebenenfalls in Gegenwart eines Säurebindemittels umsetzt.

5. Herbizide Mittel, gekennzeichnet durch einen Gehalt am mindestens einem Thiazolylderivat der allgemeinen Formel (I) gemäß den Ansprüchen 1 bis 4.

6. Verfahren zur Bekämpfung von Unkräutern, dadurch gekennzeichnet, daß man Thiazolylderivate der allgemeinen Formel (I) gemäß den Ansprüchen 1 bis 4 auf die Unkräuter und/oder ihren Lebensraum einwirken läßt.

7. Verwendung von Thiazolylderivaten der Formel (I) gemäß den Ansprüchen 1 bis 4, zur Bekämpfung von Unkräutern.

8. Verfahren zur Herstellung von herbiziden Mitteln, dadurch gekennzeichnet, daß man Thiazolylderivate der allgemeinen Formel (I) gemäß den Ansprüchen 1 bis 4 mit Streckmitteln und/oder oberflächenaktiven Substanzen vermischt.

Europäisches Patentamt

# EUROPÄISCHER RECHERCHENBERICHT

Nummer der Anmeldung: EP 88102978.9

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | CHEMICAL ABSTRACTS, Band 105, Nr. 23, 8. Dezember 1986, Columbus, Ohio, USA<br>NIPPON SODA CO. "5-Nitro-4-trifluoromethylthiazoles and fungicides"<br>Seite 595, Spalte 1, Zusammenfassung-Nr. 208 867g<br>& Jpn. Kokai Tokkyo Koho JP 61,83,174 [86,83,174]<br>-- | 1,4 | C 07 D 277/34<br>C 07 D 277/36<br>C 07 D 417/12<br>A 01 N 43/78 |
| A | CHEMICAL ABSTRACTS, Band 104, Nr. 7, 17. Februar 1986, Columbus, Ohio, USA<br>ISHIHARA SANGYO KAISHA LTD. "Substituted benzenesulfonamides containing hetero cyclic groups"<br>Seite 525, Spalte 1, Zusammenfassung-Nr. 50 896h<br>& Jpn. Kokai Tokkyo Koho JP 60,28,977 [85,28,977]<br>-- | 1,4-7 | RECHERCHIERTE SACHGEBIETE (Int. Cl.4)<br>C 07 D 277/00<br>C 07 D 417/00 |
| A | CHEMICAL ABSTRACTS, Band 100, Nr. 25, 18. Juni 1984, Columbus, Ohio, USA<br>NIHON TOKUSHU NOYAKU SEIZO K.K. "Substituted phenylurea derivatives"<br>Seite 596, Spalte 2, Zusammenfassung-Nr. 209 799v<br>& Jpn. Kokai Tokkyo Koho JP 58,216,175 [83,216,175]<br>---- | 1,4-7 | |

Der vorliegende Recherchenbericht wurde für alle Patentansprüche erstellt.

| Recherchenort | Abschlußdatum der Recherche | Prüfer |
|---|---|---|
| WIEN | 16-05-1988 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument